# EUROPEAN PATENT APPLICATION

(11) **EP 4 317 190 A1**
(43) Date of publication of application: **07.02.2024**
(21) Application number: 22775545.1
(22) Date of filing: 22.03.2022
(51) Int. Cl.: C07K 19/00, A61K 38/10, A61K 47/60, A61K 47/64, A61P 1/00, A61P 1/16, A61P 3/06, A61P 3/10, A61P 9/00, A61P 9/04, A61P 9/10, A61P 11/00, A61P 13/12, A61P 17/02, A61P 21/02, A61P 25/00, A61P 43/00, C07K 7/08, C07K 7/64, C12N 1/00

(54) **C-MET PROTEIN-BINDING PEPTIDE COMPLEX**

(30) Priority: 22.03.2021 JP 2021047949
(71) Applicant: PeptiDream Inc., Kawasaki-shi, Kanagawa 210-0821 (JP)
(72) Inventor: SUZUKI Yoshinori, Kawasaki-shi, Kanagawa 210-0821 (JP); EHARA Takeru, Kawasaki-shi, Kanagawa 210-0821 (JP); TAKUWA Masatoshi, Kawasaki-shi, Kanagawa 210-0821 (JP)
(74) Representative: van Dam, Vincent
(86) International application number: PCT/JP2022/013001
(87) International publication number: WO 2022/202761

(57) **Abstract**

[Problem] To provide a peptide complex capable of binding to c-Met protein.

[Solution] Provided is a peptide complex containing a peptide A, in which the peptide A is a peptide comprising the amino acid sequence represented by X¹-X²-X³-V-S-X⁴-D-X⁵-D-X⁶-P-R-W-X⁷-MeC (SEQ ID NO: 1) or comprises an amino acid sequence having such a structure that 1 to 3 amino acid residues are substituted, deleted, added or inserted in the amino acid sequence represented by SEQ ID NO: 1. X¹ represents A, MeA, F or MeF. X² represents V, T, E, Q or W. X³ represents A, R, Y or D. X⁴ represents F, MeF, L or MeF. X⁵ represents D, E, S, P or V X⁶ represents (S)-2-aminoheptanoic acid (Ahp), R,L-norleucine (Nle), (S)-2,7-diaminoheptanoic acid (Hty) or S. X⁷ represents S,A,L-α-aminobutanoic acid (Abu), D, Q or V

## Description

### TECHNICAL FIELD

The present invention relates to a complex containing a c-Met protein-binding peptide.

### BACKGROUND ART

In recent years, regenerative medicine, which aims to regenerate tissues damaged by disease or injury, has been attracting attention. Since regenerative medicine uses autologous cells to regenerate tissues, it has an advantage over conventional methods such as organ transplantations from other people that it is less likely to cause immune problems. In such regenerative medicine, it is necessary to culture human-derived cells such as stem cells and differentiate them into target tissues. In addition, basic research using cultured mammalian cells, especially human-derived cells, continues to be conducted to generate further improved technologies for regenerative medicine.

In regenerative medicine and research, the process of efficiently culturing and growing target cells is important.

Components of the medium play an important role in culturing of human cells, including stem cells, and growth factors (sometimes called GFs) are one of the most important components. However, growth factors are generally very expensive, and large amounts are needed to maintain cells in an undifferentiated state, for example, in stem cell-based research.

A hepatocyte growth factor receptor (HGF), which is a growth factor, and its receptor, c-Met (sometimes called c-Met or Met), are also being studied as targets for pharmaceuticals.

c-Met is a single transmembrane receptor-type tyrosine kinase. When HGF, which is a ligand, binds to c-MET, c-MET dimerizes and is activated. It is known that c-Met activation is essential for embryogenesis, organogenesis, and wound healing, and for example, binding of HGF to its receptor c-Met is known to activate relevant signal transduction pathways, specifically promote and maintain proliferation of endothelial cells and function of the endothelial cells, promote angiogenesis, and form collateral circulation from angiogenesis. Therefore, compounds with c-Met activating activity are required and studied.

In such a situation, an antibody with c-Met agonist activity is reported in Patent Literature 1. In addition, Non-Patent Literature 1 reports alternatives to HGF that are under development to date.

In recent years, use of peptide as a medium-molecular-weight compound in place of small molecules and macromolecules such as antibodies, in particular, use of cyclic peptide as a pharmaceutical composition, has been attracting attention. For example, Patent Literature 2 and Non-Patent Literature 2 describe a peptide complex which can be used as a c-Met protein agonist. The peptide complex which can be used as the c-Met protein agonist is known to promote cell proliferation and cell migration, and is expected to be used for various applications, such as pharmaceuticals and medium compositions in culturing of cells using this cell proliferation effect. For example, peptide complex is useful as a growth factor substitute to be added when cells or tissues used in regenerative medicine are cultured, an organ protectant and a regeneration promoter used during organ transplantation, and a therapeutic agent for diseases that result in decreased expression of hepatocyte growth factor.

### CITATION LIST

### PATENT LITERATURE

Patent Literature 1: Japanese Unexamined Patent Application Publication No. 2021-50793
Patent Literature 2: Japanese Patent No. 6426103

Non-Patent Literature 1: THE CHEMICAL TIMES (KANTO CHEMICAL CO., INC.), 2020, No. 2, p. 6-11 Special Feature: Development of Chemically Synthesizable Growth Factor Substitute Compounds for Regenerative Medicine Ryosuke Ueki and Shinsuke Santo
Non-Patent Literature 2: Ito, K. et. al., Nature communications, 6, Article number: 6373 (2015)

### SUMMARY OF INVENTION

### TECHNICAL PROBLEM

As mentioned above, expensive growth factors need to be added in culturing of stem cells and other cells, which has made the medical application of stem cells and other cells difficult. Since growth factors have great potential as pharmaceuticals, it is desirable to develop alternatives to growth factors that can be provided inexpensively and stably. Under such circumstances, peptide complexes that can be used as a c-Met protein agonist, containing a peptide that binds to the c-Met protein, as described in the above publications, were found. However, some of the peptide complexes described in the above publications show lower activity than human HGF. Thus, it has been desirable to develop complexes that bind to c-Met protein other than the peptide complexes.

The invention described herein is intended to solve one or more of the above.

### SOLUTION TO PROBLEM

This invention is essentially based on the finding by way of Examples that the peptide represented by SEQ ID NO: 1 binds to the c-Met protein.

This invention is also basically based on the finding by way of Examples that a peptide complex, in which the peptides are bound by a linker and designed to have a dimer structure, functions as a c-Met protein agonist.

The first invention relates to a peptide complex containing a peptide A that binds to a c-Met protein.

The peptide A is a peptide consisting of an amino acid sequence represented by X¹-X²-X³-V-S-X⁴-D-X⁵-D-X⁶-P-R-W-X⁷-MeC (SEQ ID NO: 1) or a peptide consisting of an amino acid sequence with substitution, deletion, addition, or insertion of one to three amino acids in the amino acid sequence represented by SEQ ID NO: 1 and that binds to a c-Met protein.

X¹ is an amino acid that is optionally N-alkylated.

X² is any amino acid.

X³ is any amino acid.

X⁴ is a hydrophobic amino acid that is optionally N-alkylated.

X⁵ is any amino acid.

X⁶ is an amino acid having an alkyl chain in the side chain that is optionally substituted, or S.

X⁷ is any amino acid.

### ADVANTAGEOUS EFFECTS OF INVENTION

This invention provides a complex containing a novel peptide that binds to the c-Met protein.

This invention also provides a novel peptide complex having c-Met agonist activity.

The present invention further provides, as an alternative of a growth factor to be added during culturing of cells or tissues used in regenerative medicine, a medium composition for culturing cells or tissues, containing a novel peptide complex having c-Met agonist activity. The medium composition can be used as, for example, an organ protectant or a regeneration promoter used in organ transplantation.

The present invention further provides a novel c-Met protein agonist and a pharmaceutical composition containing the c-Met protein agonist. They are useful as a therapeutic drug for diseases that result in decreased expression of hepatocyte growth factor (HGF).

The present invention further provides a pharmaceutical composition containing a novel c-Met protein agonist that promotes cell proliferation.

### BRIEF DESCRIPTION OF DRAWINGS

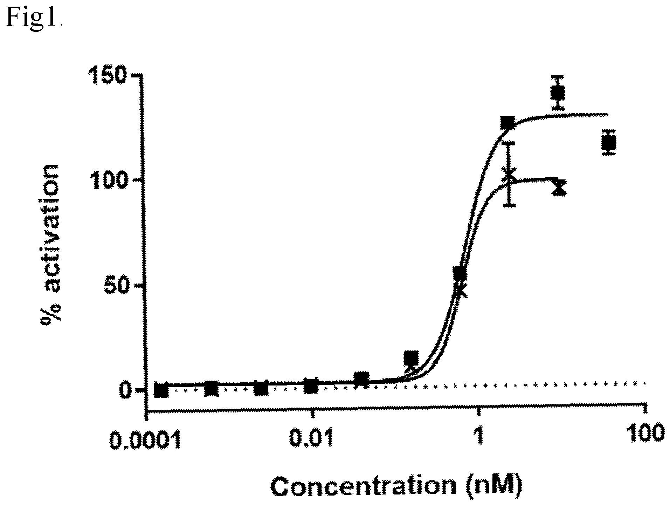

FIG. 1 shows results of measurement of c-Met agonist activity by phospho-c-Met AlphaLISA assay using a peptide complex of the present invention and human HGF. In FIG. 1, the black square indicates a c-Met agonist activity peptide complex (a complex (GFs_c-Met-00014336-PEG13 dimer; complex No. 49 in Table 4) of a peptide (SEQ ID NO: 34) and a linker (SEQ ID NO: 37)), and X indicates the human HGF. The horizontal axis indicates the concentration (nM) of the peptide complex or the human HGF, and the vertical axis indicates a relative value of an activation signal when the maximum value of the activation signal induced by the human HGF is 100.

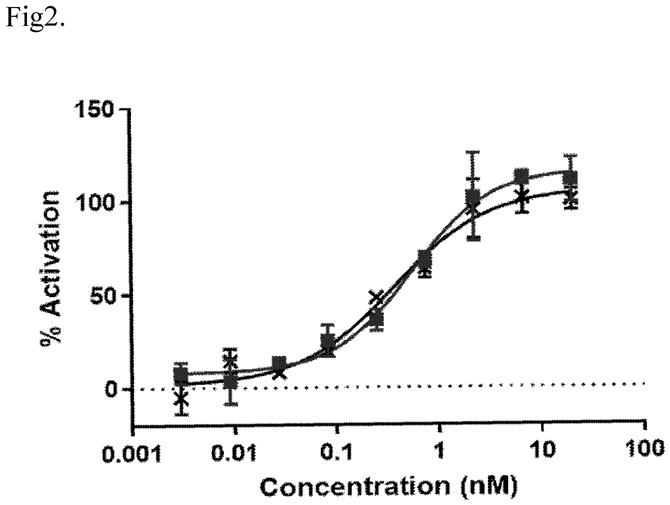

FIG. 2 shows results of measurement of c-Met agonist activity by evaluation of HUVEC cell proliferation using the peptide complex of the present invention and the human HGF. In FIG. 2, the black square indicates a c-Met agonist activity peptide complex (a complex (GFs_c-Met-00014336-PEG13 dimer; complex No. 49 in Table 4) of a peptide (SEQ ID NO: 34) and a linker (SEQ ID NO: 37)), and X indicates the human HGF. The horizontal axis indicates the concentration (nM) of the peptide complex or the human HGF, and the vertical axis indicates a relative value of an activation signal when the maximum value of the activation signal induced by the human HGF is 100.
FIG. 3 shows results obtained on the third day in a tube formation assay for renal proximal tubule epithelial cells using the peptide complex of the present invention, the human HGF, and the human epidermal growth factor (EGF).
FIG. 4 shows results obtained on the eighth day in the tube formation assay for renal proximal tubule epithelial cells using the peptide complex of the present invention, the human HGF, and the human epidermal growth factor (EGF). In FIG. 4, a black arrow indicates tube formation with branching.
FIG. 5 shows results of the human Phospho-RTK array assay evaluation using the peptide complex of the present invention and the human HGF. In FIG. 5, (1) shows an array map, and (2) shows evaluation results. In FIG. 5, black squares indicate wells in which antibodies against HGFR are fixed.

### DESCRIPTION OF EMBODIMENTS

Embodiments of the present invention will be described below. The present invention is not limited to the embodiments described below, but includes modifications made as appropriate based on the following embodiments within a scope obvious to those skilled in the art.

The first invention relates to a peptide complex containing a peptide A that binds to a c-Met protein.

### c-Met Protein

The c-Met protein is a hepatocyte growth factor (HGF) receptor and has tyrosine kinase activity. The c-Met protein is a transmembrane receptor consisting of a α-subunit and a β-subunit bound by a disulfide bond. In vivo, c-Met protein is dimerized by HGF binding, followed by autophosphorylation and activation of various signal transductions. As a result, activation of signal transductions of MAPK pathway and Akt pathway promotes cell proliferation, while induction of cell apoptosis is inhibited. If this function can be enhanced to promote cell proliferation and cell migration, it may facilitate the production of cell formulations for regenerative medicine and curing of intractable organ diseases such as hepatic cirrhosis.

The c-Met protein is also referred to as c-Met, MET, or HGFR. The GenBank accession number of the human c-Met protein is NP_000236, and that of the mouse c-Met protein is NP_032617.

### Hepatocyte Growth Factor (HGF)

The hepatocyte growth factor (HGF) is a multifunctional cytokine that functions as a growth factor for a wide range of tissues and cell strains and has a heterodimeric structure consisting of a heavy chain with a molecular weight of approximately 60000 and a light chain with a molecular weight of approximately 35000 which are disulfide-bound. HGF is known to promote proliferation of epithelial cells, endothelial cells, and mesenchymal cells, and has other functions such as induction of morphogenesis, enhancement of cell motility, anti-apoptosis, and angiogenic effects. The GenBank accession number of the human HGF is NP_000592, and that of the mouse HGF is NP_001276387. HGF is preferably human HGF, and indicates human HGF unless otherwise noted herein.

### Binding with c-Met Protein

Binding to c-Met protein means that the peptide or peptide complex binds to the c-Met protein. Whether or not the peptide or the peptide complex is bound to the c-Met protein can be measured by known methods for measuring intermolecular binding, such as any suitable methods known per se, including surface plasmon resonance (SPR) assay, Scatchard analysis and/or competitive binding assays such as radioimmunoassays (RIA), enzyme immunoassays (EIA) and sandwich competitive assays, and different variants thereof that are known per se in this art. Preferably, the measurement is an evaluation by surface plasmon resonance (SPR) spectroscopy, as described, for example, in Japanese Patent No. 6426103 (Patent Literature 2). Since the compound with c-Met agonist activity exhibits c-Met agonist activity by binding to the c-Met protein, it is possible to evaluate the c-Met agonist activity, i.e., it is possible to indirectly evaluate whether or not the peptide or the peptide complex has been bound to the c-Met protein based on the presence or absence of the c-Met agonist activity. It can be said that the complex binds to the c-Met protein if part or all of the complex is capable of binding to the c-Met protein. For example, when the complex contains peptide A, the moiety of the peptide A in the complex may be the binding site for the c-Met protein, and other moieties in the complex may also bind to the c-Met protein.

### c-Met Agonist Activity

The c-Met agonist activity refers to activity for binding to the c-Met protein and exhibits effects similar to those in HGF. Whether or not the peptide or peptide complex has c-Met agonist activity can be measured by a known method, and for example, the c-Met agonist activity can be evaluated using phospho-c-Met AlphaLISA assay or the HUVEC cell proliferation test, as shown in the Examples. The phosphorylation ability of c-Met can also be evaluated using the ELISA method described in Japanese Patent No. 6426103 (Patent Literature 2).

### Disease Cured by Peptide Complex having c-Met Agonist Activity

Examples of the disease cured by the peptide complex having c-Met agonist activity include ischemic heart disease, acute hepatitis, fulminant hepatitis, hepatic cirrhosis, biliary atresia, fatty liver, acute renal failure, chronic renal failure, diabetic nephropathy, acute pneumonia, pulmonary fibrosis, vascular disease, myocardial infarction, dilated cardiomyopathy, skin ulcer, cerebral infarction, arteriosclerosis obliterans, gastric ulcer, and amyotrophic lateral sclerosis, and the peptide complex can be used to treat these diseases.

### Peptide Complex

The peptide complex contains a peptide A that binds to a c-Met protein. The peptide complex contains one or more of the peptide A. The peptide complex preferably contains two of the peptide A. Examples of the peptide complex containing one peptide A are not limited, but such a peptide complex is a peptide complex containing peptide A and a substance (payload) to be delivered to the c-Met protein, such as a known pharmaceutical composition, or a peptide complex containing peptide A and a composition which can be a marker such as a fluorescent protein. In the case of a complex of a known pharmaceutical composition and peptide A, the binding ability of peptide A to the c-Met protein can be used to deliver the desired pharmaceutical composition to the c-Met protein.

The substance to be delivered to the c-Met protein is not particularly limited, and can be any substance desired by those skilled in the art. Examples of the substance include, but not limited to, following substances.

Compound: The compound includes not only low-molecular-weight compounds and medium-molecular-weight compounds, but also any compounds that can be introduced by the cytosis mechanism of cells. Examples include known low-molecular-weight agents.

Peptide: The peptide may be a peptide that binds to a target in the body and exhibits some effects, such as a cyclic peptide.

RI: RI may be any compound that can be labeled with a radioisotope, such as a radioisotope-labeled small- or medium-molecular-weight compound or antibody. For example, RI includes compounds for PET tests.

Protein: The protein may be any protein that exhibits a useful function in the body, such as antibodies and enzymes. For example, the protein includes enzymes used in enzyme replacement therapy.

Nucleic acid: The nucleic acid may be any base sequences such as DNA and RNA. For example, the nucleic acid includes nucleic acid medicines.

DDS: DDS may be any DDS molecules such as liposomes and micelles. The DDS molecules may further contain compounds such as pharmaceuticals inside, or complexes thereof described above.

The peptide complex can be, for example, a peptide complex including (1) a first peptide, (2) a second peptide, and (3) a linker connecting the first peptide and the second peptide.

The peptide complex may be consisting only of (1) a first peptide, (2) a second peptide, and (3) a linker connecting the first peptide and the second peptide.

In the peptide complex, at least one of the first peptide or the second peptide is a peptide A.

The first peptide and the second peptide may be identical to or different from each other, but is preferably the peptide A.

That is, the peptide complex may be a heterodimer or homodimer. In the heterodimer, the first peptide and the second peptide are different peptides (e.g., peptides A with different amino acid sequences). In the homodimer, the first peptide and the second peptide are the same peptide A. The peptide complex is preferably a homodimer.

It is preferred that in the peptide complex, the first peptide and the second peptide are the same peptide A, and the C-terminals of the first peptide and the second peptide are bound to the linker. Specifically, the first peptide and the second peptide are preferably each a cyclic peptide.

### Peptide A

Not only the complex including peptide A, but also the peptide A itself are described herein.

The peptide A is
(1) a peptide that binds to a c-Met protein consisting of an amino acid sequence represented by X¹-X²-X³-V-S-X⁴-D-X⁵-D-X⁶-P-R-W-X⁷-MeC (SEQ ID NO: 1) or
(2) a peptide that binds to a c-Met protein consisting of an amino acid sequence with substitution, deletion, addition, or insertion of one to three (one, two, or three) amino acids in the amino acid sequence represented by SEQ ID NO: 1. In the peptide of (2), V at 4th position, S at 5th position, D at 7th position, D at 9th position, P at 11th position, R at 12th position, W at 13th position, and MeC at 15th position in SEQ ID NO: 1 are preferably maintained.

X¹ is an amino acid that is optionally N-alkylated, and preferably A that is optionally N-methylated or F that is optionally N-methylated. X¹ is more preferably, MeF (N-methylated F).

X² is any amino acid, preferably a hydrophilic amino acid, an aliphatic, branched amino acid, or an aromatic amino acid, and more preferably V, T, E, Q, or W. X² is most preferably T.

X³ is any amino acid such as a hydrophilic amino acid, an aliphatic amino acid, or an aromatic amino acid, and more preferably A, R, Y, or D. X³ is most preferably A.

X⁴ is a hydrophobic amino acid that is optionally N-alkylated, preferably a hydrophobic amino acid that is optionally N-methylated, and more preferably F that is optionally N-methylated or L that is optionally N-methylated. X⁴ is most preferably MeF (N-methylated F).

X⁵ is any amino acid, preferably a hydrophilic amino acid, an aliphatic, branched amino acid, or P, and more preferably D, E, S, P, or V X⁵ is most preferably E.

X⁶ is an amino acid having an alkyl chain in the side chain that is optionally substituted, or S, preferably an (S)-2-aminoheptanoic acid (Ahp), an R, L-norleucine (Nle), an (S)-2,7-diaminoheptanoic acid (Hty), or S. X⁶ is more preferably Ahp.

X⁷ is any amino acid, preferably a hydrophilic amino acid or an aliphatic amino acid, and more preferably S, A, L-α-aminobutanoic acid (Abu), D, Q, or V. X⁷ is most preferably S.

The "amino acid that is optionally N-alkylated" means an N-alkylamino acid that is an amino acid having an alkyl group on the nitrogen forming a peptide bond, or an amino acid having no alkyl group. Examples of the N-alkylamino acid include N-butylamino acid, N-ethylamino acid, and N-methylamino acid. "Optionally N-methylated" means containing a N-methylated amino acid. For example, A that is optionally N-methylated means alanine (A) or N-methylalanine (MeA).

The "amino acid having an alkyl chain in the side chain that is optionally substituted" includes an amino acid having an alkyl chain in the side chain, for example, amino acids belonging to the group of aliphatic amino acid and amino acids in which terminals of functional groups in their side chains of these amino acids are substituted with functional groups, and is preferably an amino acid having an alkyl group with 5 or more carbon atoms. Examples thereof include Ahp, Nle, and Hty.

A preferred example of the peptide A is
(1) a peptide consisting of an amino acid sequence represented by MeF-T-A-V-S-MeF-D-E-D-Ahp-P-R-W-S-MeC (SEQ ID NO: 34) or
(2) a peptide that binds to a c-Met protein consisting of an amino acid sequence with substitution, deletion, addition, or insertion of one to three amino acids in the amino acid sequence represented by SEQ ID NO: 34.

### Conservative Amino Acid Substitution

When one, two, or three amino-acid residues are substituted, deleted, added, or inserted from a particular sequence, it is preferred that conservative amino acid substitution is performed. The "conservative amino acid substitution" means a substitution with a functionally equivalent or similar amino acid. The conservative amino acid substitution in peptide causes static changes in the amino acid sequence of the peptide. For example, one or more amino acids with similar polarity act in a functionally equivalent manner to produce a static change in the amino acid sequence of the peptide. In general, a substitution within the group can be considered conservative in structure and function. However, as is obvious to those skilled in the art, the role played by a particular amino-acid residue can be determined by its implications in the three-dimensional structure of the molecule containing that amino acid. For example, a cysteine residue can take a less polar, oxidized (disulfide) form compared to the reduced (thiol) form. The long aliphatic moiety of the arginine side chain can constitute a structurally and functionally important feature. The side chain containing an aromatic ring (such as tryptophan, tyrosine, and phenylalanine) can also contribute to ion-aromatic or cation-pi interaction. In such a case, substitution of an amino acids with these side chains with an amino acid belonging to acidic or nonpolar group can be structurally and functionally conservative. Residues such as proline, glycine, and cysteine (in disulfide form) can have a direct effect on the steric structure of the main chain and often cannot be replaced without structural distortion.

The conservative amino acid substitution includes specific substitutions based on the side chain similarity (L. Lehninger, Biochemistry, 2nd edition, pp. 73-75, Worth Publisher, New York New York (1975)) and typical substitutions, as shown below.

The conservative amino acid substitution is preferably, for example, a substitution with an amino acid belonging to the same group to which an amino acid belongs in the group of natural amino acids classified based on the common side-chain properties, as follows.

Hydrophobic (also referred to as non-polar) amino acid: The hydrophobic amino acid is an amino acid that is hydrophobic (non-polar) and includes alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply M").

The hydrophobic amino acid can be classified into the following groups.

Aliphatic amino acid: The aliphatic amino acid is an amino acid having fatty acid or hydrogen in the side chain, and includes Ala, Gly, Val, Ile, and Leu.

Aliphatic, branched amino acid: The aliphatic, branched amino acid is an amino acid having a branched fatty acid in the side chain, and includes Val, Ile, and Leu.

Aromatic amino acid: The aromatic amino acid is an amino acid having an aromatic ring in the side chain, and includes Trp, Tyr, and Phe. Hydrophilic (also referred to as polar) amino acid: The hydrophilic amino acid is an amino acid that is hydrophilic (polar), and includes serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("Lys" or simply "K"), arginine ("Arg" or simply "R"), and histidine ("His" or simply "H").

The hydrophilic amino acid can be classified into the following groups.

Acidic amino acid: The acidic amino acid is an amino acid with an acidic side chain, and includes Asp and Glu.

Basic amino acid: The basic amino acid is an amino acid with a basic side chain, and includes Lys, Arg, and His.

Neutral amino acid: The neutral amino acid is an amino acid with a neutral side chain, and includes Ser, Thr, Asn, Gln, and Cys.

Gly and Pro can be classified into an "amino acid which affects the direction of the main chain," and an amino acid having a sulfur molecule in the side chain, Cys, and Met can be classified into a "sulfur-containing amino acid."

The "amino acid" herein includes not only a natural amino acid but also a non-natural amino acid. Examples of the non-natural amino acid include N-alkylamino acid in which the above-mentioned natural amino acid is N-alkylated, and an amino acid in which nitrogen which forms a peptide bond is modified with a branched or unbranched lower (e.g., C1 to C5, preferably C1 to C3, and more preferably C1) alkyl group. The N-alkylamino acid is preferably N-ethylamino acid, N-butylamino acid, or N-methylamino acid, and more preferably N-methylamino acid. Non-natural amino acid includes chemically modified amino acids such as D-type amino acid (also called D-amino acid), β-amino acid, γ-amino acid, amino acid variants, and amino acid derivatives; and amino acids which are not constituent materials of protein in vivo such as norleucine and ornithine. The non-natural amino acid further includes an amino acid in which a functional group is further added to the side chain of natural amino acid or which is substituted with another functional group, (such as an amino acid with a substitution or addition in a moiety of arylene group or alkylene group of the side chain, an amino acid with increased carbon atoms of arylene group, alkylene group, or alkyl group of the side chain, an amino acid with a substitution in aromatic ring of the side chain, and a heterocyclized or fused amino acid).

The addition or substitution of a functional group or other structure to the side chain of a natural amino acid can impart properties different from those of the natural amino acid. For example, A4p is alanine with a piperidyl group attached to the side chain, and the addition of the piperidyl group gives it the basic polarity, unlike alanine, which belongs to the group of non-polar amino acid.

In other words, a non-natural amino acid with similar side-chain properties can be included in the above-mentioned group of natural amino acids based on their common side-chain properties. For example, N-methylarginine (MeR), which is an N-methylated amino acid of arginine, belonging to basic amino acid, is a non-natural amino acid but can be classified as a basic amino acid because it exhibits basic properties. Thus, the non-natural amino acid that exhibits similar side-chain properties to an amino acid can also be included as targets for conservative amino acid substitution.

Non-limiting examples of the non-natural amino acid include N-methylamino acid, Ahp, Nle, Hty, and Abu. For example, Ahp, Nle, Abu, and Hty can be classified into hydrophobic amino acid, Ahp, Nle, and Abu can be classified into aliphatic amino acid, and Hty can be classified into aromatic amino acid. N-methylamino acid can be classified into N-alkylamino acid or classified according to the side-chain properties of the original amino acid that is not N-methylated.

In particular, the peptide A is preferably a peptide having an amino acid sequence of any of SEQ ID Nos: 2 to 34. Among them, the peptide A is preferably a peptide having an amino acid sequence of SEQ ID NO: 34.

The peptide A is preferably a cyclic peptide.

The term "peptide" refers to a structure consisting of multiple contiguous amino acids, and encompasses polypeptides and proteins. In this application, the term "amino acid" includes not only naturally derived amino acids (natural amino acids) but also amino acids that do not exist naturally (non-natural amino acids).

In the present application, the peptide of the present invention includes peptides with cyclic portions formed by postsynthetic cyclization, peptides obtained by further chemical modification of the peptides, and complexes of peptides and substances bound to the peptides.

In this specification, some amino acids may be modified for cyclization of peptide. The peptide encompasses peptides containing amino acids modified as described above. An example of modification for cyclization is addition of a chloroacetyl group to amino acid at the N-terminal, which binds to a cysteine residue in the peptide to form a ring. The peptide of the present application further encompasses various (natural/non-natural) amino acids with addition of chloroacetyl groups.

The cyclic peptide refers to one in which two amino acids in a peptide are bound to form a ring in whole or in part. In this application, the cyclic peptide encompasses those in which amino acids in peptides form a cross-linked structure, those in which a cyclic structure is formed by lactam ring formation or macrocyclization reaction, and those having a lasso peptide-like structure are also included. In other words, in the present application, the cyclic peptide may have a linear chain portion, as long as a portion of the peptide forms a cyclic structure.

Peptides generally have poor metabolic stability in vivo, and their sizes are large, which makes them difficult to permeate cell membranes. In order to address such a problem, cyclization of peptides has been used. It has been suggested that cyclization of peptides improves protease resistance, improves metabolic stability, and limits conformational changes, thereby increasing rigidity and improving membrane permeability and affinity for target proteins.

Cyclization of peptides can be performed according to known methods. Although not limited to this, for example, by designing the peptide to contain two or more cysteine residues, a cyclic structure can be formed by a disulfide bond after translation. The cyclization can be achieved also by synthesizing a peptide having a chloroacetyl group at the N-terminal and placing a cysteine residue in the peptide using the genetic code reprogramming technique according to the method of Goto et al (Y Goto, et al. ACS Chem. Biol. 3 120-129 (2008)). This causes spontaneous nucleophilic attack of the mercapto group to the chloroacetyl group after translation, and the peptide is circularized by a thioether bond. Other combinations of amino acids that binds to form a ring may be placed within the peptide to achieve cyclization by the genetic code reprogramming technique. Alternatively, a peptide having cycloamide at the N terminal may be synthesized and circularized by placing an L-2-amino adipic acid residue in the peptide and binding between them. Thus, any known cyclization method can be used without particular limitations.

### Peptide Length of Peptide A

The peptide length (the number of amide bonds) of the peptide A is not particularly limited, but the total number of amino-acid residues (if the substance bound to the peptide or the linker that binds the substance to the peptide contains amino acids, those amino acids are not included) is preferably within 20 residues. The total number of amino acid residues is preferably 6 or more, 7 or more, 8 or more, 9 or more, 10 or more, 11 or more, and 19 or less, 18 or less, 17 or less, 16 or less, 15 or less.

### Nucleic Acid Encoding Peptide A

A nucleic acid encoding the peptide A (c-Met protein-binding peptide) is also described herein. The "nucleic acid" herein may be natural or non-natural nucleic acid, and includes DNA, RNA, and chimeras thereof, but is not limited thereto.

### Linker

The type and the length of the linker used in the peptide complex are not particularly limited as long as the peptide complex can bind to the c-Met protein. Examples of the linker include an amino acid linker (a peptide linker), a chemical linker, a fatty acid linker, a nucleic acid linker, and a carbohydrate chain linker, and the linker may be a complex of the chemical linker and the peptide linker. Examples of the chemical linker include a polyethyleneglycol (PEG) linker. The linker may also be a fatty acid linker containing a divalent chemical moiety derived from a fatty acid. The amino acid (peptide) linker is a linker containing at least one any amino acid, and can be, for example, glycine-rich peptide such as a peptide having a sequence [Gly-Gly-Gly-Gly-Ser]n (in which n is 1, 2, 3, 4, 5, or 6) including those described in U.S. Patent No. 7,271,149, and serine-rich peptide linker described in U.S. Patent No. 5,525,491. These linkers can be bound to the c-Met protein-binding peptide by known methods or equivalent method thereof. For example, a linker is bound to the c-Met protein-binding peptide by binding the linker to a Cys residue at the terminal of the c-Met protein-binding peptide. The linker can also be bound to an amino acid contained in the c-Met protein-binding peptide other than the C-terminal.

The linker is, for example, preferably a PEG linker. The PEG linker is a linker containing polyethylene glycol (PEG) or a polyethylene glycol (PEG) derivative. The PEG linker may contain amino acid. The PEG linker preferably contains 8 or more PEG molecular units. Specific examples of the linker include: (1) a linker having a sequence represented by any of SEQ ID Nos: 35 to 41; (2) a linker having a sequence with substitution, deletion, addition, or insertion of one to three amino acids in the sequence represented by any of SEQ ID Nos: 35 to 41 (the amino acid is not particularly limited as long as it can be used as a linker, but includes, for example, S, G, and K and one or more of the amino acids).

The peptide A, the linker, and the peptide complex can be prepared by known method (e.g., Japanese Patent No. 6426103 (Patent Literature 2)) or by modifying the known method as appropriate.

### c-Met Protein Agonist

The peptide complex has an HGF-like function through promoting autophosphorylation by multimerization of c-Met protein. Therefore, the peptide complex is useful as a c-Met agonist (agonist) and a pharmaceutical composition containing the c-Met protein agonist.

### Pharmaceutical Composition

A pharmaceutical composition containing the peptide complex and a pharmaceutically acceptable carrier is also described herein. Among the peptide complexes, a pharmaceutical composition containing a c-Met protein agonist is used to treat or prevent a disease selected from the group consisting of ischemic heart disease, acute hepatitis, fulminant hepatitis, hepatic cirrhosis, biliary atresia, fatty liver, acute renal failure, chronic renal failure, diabetic nephropathy, acute pneumonia, pulmonary fibrosis, vascular disease, myocardial infarction, dilated cardiomyopathy, skin ulcer, cerebral infarction, arteriosclerosis obliterans, gastric ulcer, and amyotrophic lateral sclerosis.

The pharmaceutical composition according to the present invention contains, as an active ingredient, the peptide complex according to the present invention. Since the pharmaceutical composition has an HGF-like function, it is useful for promoting cell proliferation, promoting cell migration, inhibiting apoptosis, inducing morphogenesis, angiogenesis, and regenerating and protecting tissues and organs, and is used as a therapeutic or preventive agent for diseases related to these. Examples of the disease include acute hepatitis, fulminant hepatitis, hepatic cirrhosis, biliary atresia, fatty liver, acute renal failure, chronic renal failure, diabetic nephropathy, acute pneumonia, pulmonary fibrosis, vascular disease, myocardial infarction, dilated cardiomyopathy, skin ulcer, cerebral infarction, arteriosclerosis obliterans, gastric ulcer, and amyotrophic lateral sclerosis.

The administration route of the pharmaceutical composition is not particularly limited, and may be administered orally or parenterally. Examples of the parenteral administration include: administration by injection such as intramuscular injection, intravenous injection, and subcutaneous injection; transdermal administration; and transmucosal administration (transnasal administration, oral administration, ocular administration, pulmonary administration, vaginal administration, and rectal administration).

Peptides in the pharmaceutical composition can be modified in various ways in view of their metabolic and excretory properties. For example, polyethylene glycol (PEG) or a carbohydrate chain can be added to polypeptides to increase their residence time in blood and decrease their antigenicity. In addition, biodegradable polymer compounds such as poly(lactic-co-glycolic acid) (PLGA), porous hydroxyapatite, liposomes, surface-modified liposomes, and emulsions, nanoparticles, and nanospheres prepared from unsaturated fatty acids may be used as a sustained release substrate, and the polypeptide may be encapsulated therein. When the pharmaceutical composition is administered transdermally, a weak electric current can be applied to the skin surface to penetrate the stratum corneum (iontophoresis method).

In the pharmaceutical composition, active ingredients may be used as they are, or a pharmaceutically acceptable carrier, excipient, additive, or the like may be added to the pharmaceutical composition to formulate. Examples of the dosage form include a liquid (e.g., an injection), a dispersant, a suspension, a tablet, a pill, a powder, a suppository, a powdered medicine, a fine granule, a granule, a capsule, a syrup, a troche, an inhalante, an ointment, eye drops, nose drops, ear drops, and a plaster.

The formulation may be performed in the usual manner using, for example, an excipient, a binder, a disintegrant, a lubricant, a solvent, a solubilizer, a colorant, a flavor, a stabilizing agent, an emulsifier, a sorbefacient, a surfactant, a pH adjuster, a preservative, an antioxidant, and the like, as appropriate.

Examples of components used for the formulation include purified water, saline, a phosphate buffer solution, dextrose, glycerol, pharmaceutically acceptable organic solvents such as ethanol, animal and vegetable oils, lactose, mannitol, glucose, sorbitol, crystalline cellulose, hydroxypropyl cellulose, starch, cornstarch, anhydrous silicic acid, aluminum magnesium silicate, collagen, polyvinyl alcohol, polyvinylpyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethyleneglycol, diglycerine, glycerin, propylene glycol, vaseline, paraffin, octyldodecyl myristate, isopropyl myristate, higher alcohol, stearyl alcohol, stearic acid, and human serum albumin, but the components are not limited to these.

The sorbefacient that improves absorption of poorly absorbable drugs can be, for example, a surfactant such as polyoxyethylene lauryl ethers, sodium lauryl sulfate, and saponin; a bile salt such as glycocholic acid, deoxycholic acid, and taurocholic acid; a chelating agent such as EDTA and salicylic acids; fatty acids such as caproic acid, capric acid, lauric acid, oleic acid, linoleic acid, and mixed micelles; enamine derivatives, N-acyl collagen peptides, N-acylamino acids, cyclodextrins, chitosan, and nitric oxide donors.

The pill or tablet can also be coated with a saccharide, gastric, or enteric soluble substance. The injection may contain distilled water for injection, a saline solution, propylene glycol, polyethylene glycol, a vegetable oil, and alcohols. The injection may further contain a humectant, an emulsifier, a dispersant, a stabilizing agent, a solvent, a solubilizer, a preservative, and the like.

The pharmaceutical composition of the present invention may be administered in combination with other pharmaceuticals or treatments useful for the above diseases.

When the pharmaceutical composition of the present invention is administered to mammals (e.g., humans, mice, rats, guinea pigs, rabbits, dogs, horses, monkeys, pigs, sheep, etc.), especially humans, the dose depends on the symptoms, age, sex, weight, and sensitivity difference of the subject, administration method, administration interval, type of active ingredient, and type of formulation. The dose is, for example, 30 µg to 1000 mg, 100 µg to 500 mg, or 100 µg to 100 mg for one or several doses. For injection administration, 1 µg/kg to 3000 µg/kg or 3 µg/kg to 1000 µg/kg may be administered for one or several doses, depending on the body weight of the subject.

### Treatment Method

This specification also provides a method of treating the various diseases described above, comprising administering, to a subject (e.g., a mammal or patient), an effective amount of a peptide complex or an effective amount of a pharmaceutical composition. This specification also describes the use of peptide A or the peptide complex, in the manufacture of the pharmaceutical composition, as well as the peptide A, and the method of manufacturing the pharmaceutical composition using the peptide complex.

### Culture Medium Additive and Medium

This specification also provides a culture medium additive containing the peptide complex and a culture medium containing the peptide complex. The culture medium additive is used to culture mammal-derived cells or tissues. Examples of cells to be cultured include, but are not limited to, somatic cells, germ cells, and pluripotent stem cells, which are cells that have the ability to differentiate into all tissues and cells that constitute a biological body. Examples of the pluripotent stem cells include ES cells, sperm stem cells, pluripotent germline stem cells, embryonic germline cells, iPS cells, cultured fibroblasts, or cells derived from bone marrow stem cells. They may be fusion cells of stem cells and somatic cells, pluripotent stem cells induced and selected by stress or cell stimulation, or pluripotent stem cells established by culturing early embryos created by nuclear transfer of somatic cell nuclei. Examples of tissues to be cultured include tissues differentiated from cells and tissues removed from the body. For example, this culture medium additive can be used to protect regenerated tissue or organs for organ transplantation.

The cells or tissues are preferably cells or tissues derived from primate, such as human, monkey, and chimpanzee, and more preferably of human.

The medium is not particularly limited as long as it is a medium for culturing cells or tissues, but is preferably a medium to which human HGF is added. The medium may be a serum medium, preferably a serum-free medium or low serum medium.

The culture medium additive may be in a solution form or a dried solid form (e.g., solid, powder). If the culture medium is in the solution form, it may be used as it is as a culture medium, or it may be diluted with a solvent and is caused to contain the additives as needed, and then used as a culture medium. Examples of a solvent used for the dilution include water, buffer solutions, saline solution, and culture media used for various cultures of cells and tissues, which may be used alone or in combination of two or more of them.

If the culture medium additive is in the dried solid form, it may be dissolved in a solvent such as water, a buffer solution, a saline solution, and media used for various cultures of cells and tissues and is caused to contain the additives as needed, and is then used as a culture medium.

The content of the peptide complex of the present invention in the medium for culturing cells or tissues or in the medium for cells obtained therefrom can be set arbitrarily by those skilled in the art, but for example, the final concentration may be about 0.01 nmol/L to about 10000 nmol/L, preferably about 0.1 nmol/L to about 1000 nmol/L, more preferably about 0.5 nmol/L to about 1000 nmol/L, and yet more preferably about 1 nmol/L to about 100 nmol/L relative to the total amount of the composition or the medium.

### DDS Carrier

The peptide A has a property of binding to the c-Met protein. Therefore, the peptide A or a peptide complex containing the peptide A can function as a DDS carrier (drug delivery carrier) or drug delivery complex by binding with known substances such as drugs, to be delivered to the c-Met protein. This specification further discloses such a DDS carrier and a drug delivery complex. The peptide A or peptide complex can be bound to a drug using known methods.

### EXAMPLES

(General) Abbreviations
General abbreviations include: Å as angstrom (unit);
BSA as bovine serum albumin;
DMSO as dimethyl sulfoxide;
DMF as dimethylformamide;
DIPEA or DIEA as N,N-diisopropylethylamine;
DODT as 3,6-dioxa-1,8-octane-dithiol;
DMEM as Dulbecco's modified Eagle's medium;
EC50 as 50% effective concentration;
EGF as epidermal growth factor;
Fmoc as 9-fluorenylmethyloxycarbonyl;
FBS as fetal bovine serum;
Fmoc-Lys(Fmoc)-OH as N²,N⁶-bis(((9H-fluoren-9-yl)methoxy)carbonyl)-L-lysine;
g as gram (unit);
HATU as O-(7-azabenzotriazol-1-yl)-N,N,N',N'-tetramethyluronium hexafluorophosphate;
HGFR as HGF receptor;
LC-MS or LC/MS as liquid chromatography mass spectrometer;
mL as milliliter (unit); M as molar (unit); µL as microliter (unit);
mM as milimolar (unit);
mg as milligram (unit);
MeCN as acetonitrile;
min as minute (unit);
mm as millimeter (unit);
nm as nanometer (unit);
REBM as renal epithelial cell basal medium;
rpm as revolutions per minute (unit);
TFA as trifluoroacetic acid; and
TIS as triisopropylsilane.

### Abbreviations (Non-Natural Amino Acids)

Abbraviations of non-natural amino acids include MeF as N-methyl-L-phenylalanine;
MeA as N-methyl-L-alanine;
Ahp as (S)-2-aminoheptanoic acid;
Nle as L-norleucine;
Hty as (S)-2-amino-4-(4-hydroxyphenyl)butanoic acid;
Abu as (S)-2-aminobutanoic acid;
MeC as N-methyl-L-cysteine;
PEG4c as 1-amino-3,6,9,12-tetraoxa-15-pentadecanoic acid;
PEG8c as 1-amino-3,6,9,12-tetraoxapentadecane-15-oic acid;
PEG12c as 1-amino-3,6,9,12,15,18,21,24,27,30,33,36-dodecaoxanonatriacontan-39-oic acid;
cPEG1c as 3,3'-oxydipropionic acid;
cPEG9c 4,7,10,13,16,19,22,25,28-nonaoxahentriacontanedioic acid;
cPEG17c as 4,7,10,13,16,19,22,25,28,31,34,37,40,43,46,49,52-heptadecaoxapentapentacondioic acid; and
OCOPEG13OCO as 3,6,9,12,15,18,21,24,27,30,33-undecaoxapentatriacontane-1,35-diylbis (bicarbonate).

### EXAMPLE 1

### Chemical Synthesis

As all raw materials, building blocks, reagents, acids, bases, solid-phase resins, and solvents used in the chemical syntheses in the following Examples are commercially available products were used as they are, or they were synthesized by those skilled in the art using organic chemical techniques. An amino acid containing a protecting group was used as it was commercially available unless otherwise noted.

Elongation of a peptide chain in the solid-phase resin was performed by using resins described in Examples as starting materials and using commonly used peptide coupling reaction conditions and Fmoc removal reaction conditions. Reaction was performed using Siro I manufactured by Biotage, which is an automated peptide synthesizer, according to the manufacturer's manual. Commonly used amino acids used herein are listed below, and the side chain protecting groups are indicated in parentheses,

Fmoc-Trp(Boc)-OH; Fmoc-Thr(tBu)-OH; Fmoc-N-Me-Gly-OH; Fmoc-Asp(OtBu)-OH; Fmoc-N-Me-Phe-OH; Fmoc-Ala-OH; Fmoc-N-Me-Ala-OH; Fmoc-His(Trt)-OH; Fmoc-Tyr(tBu)-OH; Fmoc-Val-OH; Fmoc-HydPro(tBu)-OH; Fmoc-Cys(Trt)-OH; Fmoc-Lys(Mtt)-OH; Fmoc-Ser(tBu)-OH; Fmoc-N-Me-Ser(tBu)-OH.

The resultant crude peptide was purified by reversed-phase preparative HPLC on an AutoPurification System-SQD2 single quadruple mass spectrometer available from Waters, and elution was performed while monitoring m/z ions derived from the target peptide. It was confirmed that the mass spectrum obtained in the ESI-positive scan mode and the mass spectrum including multiply charged ions calculated from the molecular formula of the target product agreed within the error range of the mass spectrometer used. The purification conditions, including the columns used, are shown in each Example.

The structure determination of the chemically synthesized peptide was checked by ESI-MS(+) in mass spectrometry, where the molecular weight was calculated by considering the amino acids used according to the target sequence and the building blocks used as necessary. The term "ESI-MS(+)" indicates electrospray ionization mass spectrometry performed in positive ion mode. The detected masses are reported in "m/z" units. Compounds with molecular weights roughly greater than 1000 were frequently detected as divalent or trivalent ions.

### EXAMPLE 2

### Identification of Peptide with c-Met Agonist Activity

The c-Met agonist peptides were targeted to Recombinant Human HGF R/c-Met Fc Chimera His-tag Protein (R&D systems) and identified by the screening methods described in International Publication WO 2014/119600, International Publication WO 2012/033154, or International Publication WO 2007/066627. For the purpose of checking whether or not the peptides actually have c-Met agonist activity, they were chemically synthesized. The peptides were synthesized as homodimers (peptide complexes), in each of which two peptides were bound by a linker. The amino acid sequences of the peptide moieties in the synthesized peptide complexes are shown in Table 1 and the linker sequences in Table 2. The synthesized peptide complexes (combinations of peptides and linkers) are shown in Tables 3 and 4.

**Table 1: Amino Acid Sequence of Peptide Moiety**

| Peptide SEQ ID No. | GF peptide number | SEQ | | | | | | | | | | | | | | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 |
| 2 | GFs_c-Met_0012p | MeF | V | Y | V | S | MeF | D | D | D | Ahp | P | R | W | S | MeC |
| 3 | GFs_c-Met_0012_17p | MeA | V | Y | V | S | MeF | D | D | D | Ahp | P | R | W | S | MeC |
| 4 | GFs_c-Met_0012_18p | F | V | Y | V | S | MeF | D | D | D | Ahp | P | R | W | S | MeC |
| 5 | GFs_c-Met_0012_19p | A | V | Y | V | S | MeF | D | D | D | Ahp | P | R | W | S | MeC |
| 6 | GFs_c-Met_0012_1p | MeF | T | Y | V | S | MeF | D | D | D | Ahp | P | R | W | S | MeC |
| 7 | GFs_c-Met_0012_2p | MeF | W | Y | V | S | MeF | D | D | D | Ahp | P | R | W | S | MeC |
| 8 | GFs_c-Met_0012_3p | MeF | V | D | V | S | MeF | D | D | D | Ahp | P | R | W | S | MeC |
| 9 | GFs_c-Met_0012_4p | MeF | V | A | V | S | MeF | D | D | D | Ahp | P | R | W | S | MeC |
| 10 | GFs_c-Met_0012_5p | MeF | V | Y | V | S | MeF | D | V | D | Ahp | P | R | W | S | MeC |
| 11 | GFs_c-Met_0012_6p | MeF | V | Y | V | S | MeF | D | P | D | Ahp | P | R | W | S | MeC |
| 12 | GFs_c-Met_0012_7p | MeF | V | Y | V | S | MeF | D | D | D | Nle | P | R | W | S | MeC |
| 13 | GFs_c-Met_0012_8p | MeF | V | Y | V | S | MeF | D | D | D | Hty | P | R | W | S | MeC |
| 14 | GFs_c-Met_0012_9p | MeF | V | Y | V | S | MeF | D | D | D | S | P | R | W | S | MeC |
| 15 | GFs_c-Met_0012_10p | MeF | V | Y | V | S | MeF | D | D | D | Ahp | P | S | W | S | MeC |
| 16 | GFs_c-Met_0012_11p | MeF | V | Y | V | S | MeF | D | D | D | Ahp | P | R | W | Abu | MeC |
| 17 | GFs_c-Met_0012_12p | MeF | V | Y | V | S | MeF | D | D | D | Ahp | P | R | W | A | MeC |

**[Table 1-2]**

| | | | | | | | | | | | | | | | | |
|---|---|---|---|---|---|---|---|---|---|---|---|---|---|---|---|---|
| 18 | GFs_c-Met_0012_13p | MeF | V | Y | V | S | MeF | D | D | D | Ahp | P | R | W | D | MeC |
| 19 | GFs_c-Met_0012_14p | MeF | V | Y | V | S | MeF | D | D | D | Ahp | P | R | W | H | MeC |
| 20 | GFs_c-Met_0012_15p | MeF | V | Y | V | S | MeF | D | D | D | Ahp | P | R | W | Q | MeC |
| 21 | GFs_c-M et_0012_16p | MeF | V | Y | V | S | MeF | D | D | D | Ahp | P | R | W | V | MeC |
| 22 | GFs_c-Met-00014295p | MeF | E | A | V | S | MeF | D | S | D | Ahp | P | R | W | A | MeC |
| 23 | GFs_c-Met-00014298p | MeF | E | A | V | S | MeF | D | P | D | Ahp | P | R | W | A | MeC |
| 24 | GFs_c-Met-00014301p | MeF | E | A | V | S | MeF | D | P | D | Ahp | P | R | W | S | MeC |
| 25 | GFs_c-Met-00014296p | MeF | Q | A | V | S | MeF | D | S | D | Ahp | P | R | W | A | MeC |
| 26 | GFs_c-Met-00014299p | MeF | Q | A | V | S | MeF | D | P | D | Ahp | P | R | W | A | MeC |
| 27 | GFs_c-Met-00014302p | MeF | Q | A | V | S | MeF | D | P | D | Ahp | P | R | W | S | MeC |
| 28 | GFs_c-Met-00014303p | MeF | T | R | V | S | MeF | D | S | D | Ahp | P | R | W | S | MeC |
| 29 | GFs_c-Met-00014306p | MeF | T | A | V | S | L | D | S | D | Ahp | P | R | W | S | MeC |
| 30 | GFs_c-Met-00014304p | MeF | T | A | V | S | MeF | D | S | D | R | P | R | W | S | MeC |
| 31 | GFs_c-Met-00014294p | MeF | T | A | V | S | MeF | D | S | D | Ahp | P | R | W | A | MeC |
| 32 | GFs_c-Met-00014297p | MeF | T | A | V | S | MeF | D | P | D | Ahp | P | R | W | A | MeC |
| 33 | GFs_c-Met-00014300p | MeF | T | A | V | S | MeF | D | P | D | Ahp | P | R | W | S | MeC |
| 34 | GFs_c-Met-00014336p | MeF | T | A | V | S | MeF | D | E | D | Ahp | P | R | W | S | MeC |

**Table 2: Linker Sequence**

| [Table 2] | | | | | |
|---|---|---|---|---|---|
| Linker SEQ ID No. | Linker | | | | |
| 35 | PEG12c | K | PEG12c | | |
| 36 | G | K | cPEG9c | K | G |
| 37 | G | K | OCOPEG13OCO | K | G |
| 38 | G | K | cPEG17c | K | G |
| 39 | PEG4c | K | PEG4c | | |
| 40 | PEG8c | K | PEG8c | | |
| 41 | G | K | cPEG1c | K | G |

**Table 3: Peptide Complex**

| [Table 3] | | | | | |
|---|---|---|---|---|---|
| **Table 3 Peptide Complex** | **Peptide Moiety** Peptide SEQ ID No. | **Linker Moiety** | **c-Met Agonist Activity** (2 or 1) | ESI (m/z) | [M+XH]X+ |
| 1 | 2 | 35 | 7 | 1300.6 | 4 |
| 2 | 3 | 35 | 1 | 1683.4 | 3 |
| 3 | 4 | 35 | 1 | 1724.9 | 3 |
| 4 | 5 | 35 | 1 | 1674.2 | 3 |
| 5 | 1 | 35 | 2 | 1792.0 | 3 |
| 6 | 8 | 35 | 2 | 1702.1 | 3 |
| 7 | 10 | 35 | 2 | 1723.7 | 3 |
| 8 | 12 | 35 | 2 | 1725.0 | 3 |
| 9 | 14 | 35 | 2 | 1281.0 | 4 |
| 10 | 15 | 35 | 1 | 1687,9 | 3 |
| 11 | 16 | 35 | 2 | 1732.9 | 3 |
| 12 | 18 | 35 | 2 | 1752.8 | 3 |
| 13 | 19 | 35 | 2 | 1767.3 | 3 |
| 14 | 20 | 35 | 2 | 1761.4 | 3 |
| 15 | 21 | 35 | 2 | 1742.6 | 3 |
| 15 | 9 | 41 | 1 | 1401.2 | 3 |

**Table 4: Peptide complex**

| [Table 4] | | | | | |
|---|---|---|---|---|---|
| Table 4 **Peptide Complex** | **Peptide Moiety** Peptide SEQ ID No. | **Linker Moiety** SEQ ID No. | **c-Met Agonist Activity** (C50; nM) | ESI (m/z) | [M+XH]X+ |
| 17 | 2 | 35 | 0.76 | 1300.6 | 4 |
| 18 | 6 | 35 | 1.31 | 1301.6 | 4 |
| 19 | 9 | 35 | 1.22 | 1254.6 | 4 |
| 20 | 11 | 35 | 2.22 | 1291.6 | 4 |
| 21 | is | 35 | 2.17 | 1325.6 | 4 |
| 22 | 17 | 35 | 2.37 | 1292.8 | 4 |
| 23 | 9 | 38 | 0.91 | 1227.3 | 4 |
| 24 | 9 | 36 | 1.27 | 1139.2 | 4 |
| 25 | 22 | 38 | 0.81 | 1220.2 | 4 |
| 26 | 22 | 36 | 0.75 | 1132.1 | 4 |
| 27 | 23 | 38 | 2.37 | 1225.2 | 4 |
| 28 | 23 | 36 | 1.24 | 1137.1 | 4 |
| 29 | 24 | 38 | 0.88 | 1233.2 | 4 |
| 30 | 24 | 36 | 0.81 | 1145.1 | 4 |
| 31 | 25 | 38 | 0.68 | 1219.7 | 4 |
| 32 | 25 | 36 | 0.51 | 1131.6 | 4 |
| 33 | 26 | 38 | 2.05 | 1224.7 | 4 |
| 34 | 26 | 36 | 1.26 | 1136.7 | 4 |
| 35 | 27 | 38 | 0.92 | 1232.8 | 4 |
| 36 | 27 | 36 | 0.73 | 1144.6 | 4 |
| 37 | 28 | 38 | 0,49 | 1266.8 | 4 |
| 38 | 28 | 36 | 0.65 | 1168.7 | 4 |
| 39 | 29 | 38 | 1.44 | 1190.3 | 4 |
| 40 | 29 | 36 | 2.13 | 1102.1 | 4 |
| 41 | 30 | 38 | 0.32 | 1140.6 | 4 |
| 42 | 30 | 36 | 0.33 | 1228.8 | 4 |
| 43 | 31 | 38 | 0.45 | 1206.2 | 4 |
| 44 | 31 | 36 | 0.33 | 1118.2 | 4 |
| 45 | 32 | 38 | 0.71 | 1211.2 | 4 |
| 46 | 32 | 36 | 0.67 | 1123.1 | 4 |
| 47 | 33 | 38 | 0.50 | 1219.2 | 4 |
| 48 | 33 | 36 | 0.35 | 1131.1 | 4 |
| 49 | 34 | 37 | 0.67 | 1177.2 | 4 |
| 50 | 34 | 38 | 0.27 | 1235.3 | 4 |
| 51 | 34 | 36 | 0.24 | 1147.1 | 4 |

### EXAMPLE 3

### Chemical Synthesis of Peptide with c-Met Agonist Activity

### [Example 3-1]

The peptide complexes listed in Table 3 were synthesized in the same manner as the synthesis of peptide complexes shown below unless there were other synthesis examples. The ESI-MS (m/z) in Table 3 indicates the ESI-MS (+) observed value, and [M+XH]X+ indicates the value of X when the number of protons added in that case is shown as (M+XH)X+.

### Synthesis of GFs c-Met 0012 7 complex (GFs c-Met 0012 7-PEG12c-K dimer: peptide complex No. 8 in Table 3)

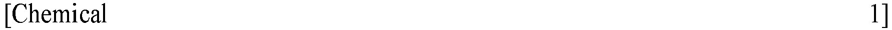

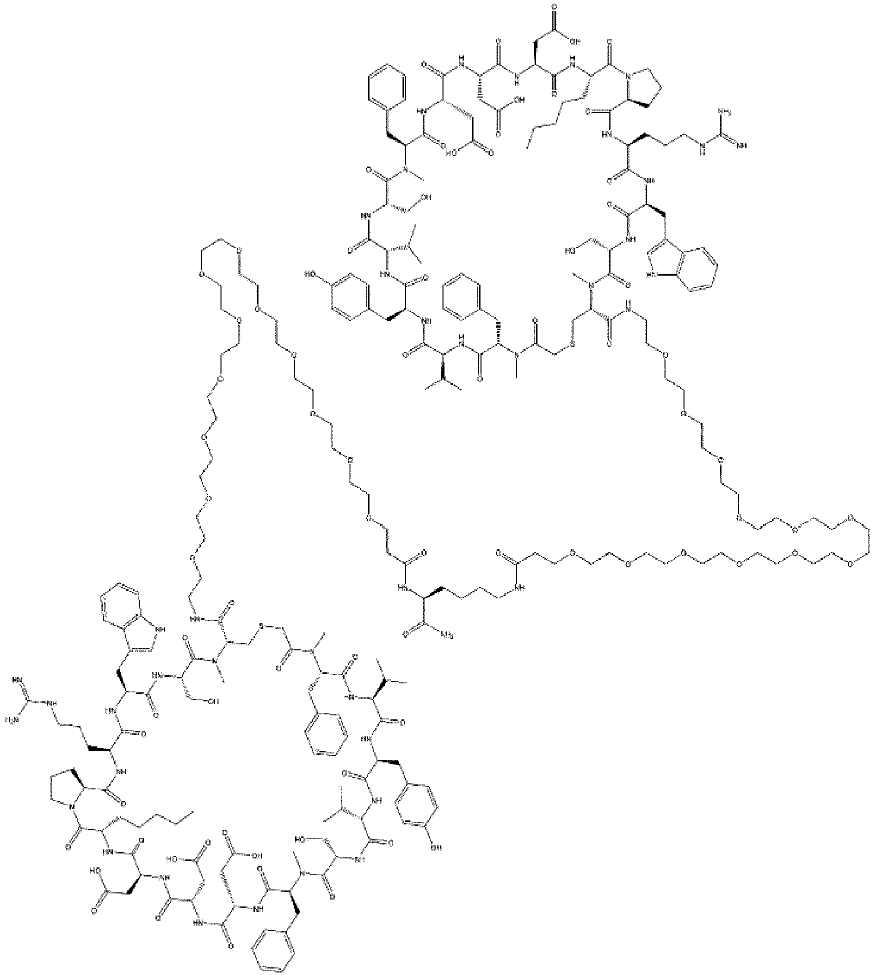

A target peptide was synthesized using NovaPEG Rink Amide resin (Merck, 0.53 mmol/g, 0.005 g), starting with removal of the Fmoc group by the general method described above. The synthesis was performed using Siro I available from Biotage as a solid-phase synthesizer, following the manufacturer's manual. The synthesis was performed by after introducing Fmoc-Lys(Fmoc)-OH into the solid-phase resin, removing both Fmoc groups and the simultaneously elongating from the two amino groups on Lys. For the introduction of each residue, Fmoc-AA/HATU/DIPEA (8.4 equivalents/7.8 equivalents/16.8 equivalents) was used per equivalent of resin. For Fmoc removal, Fmoc was reacted with a 20% piperidine solution in DMF at 25°C for 5 minutes, then the solution was removed, and a 20% piperidine solution in DMF was added again and reacted for 15 minutes. Introduction of a chloroacetyl group into the solid-phase resin containing the Fmoc-protected peptide obtained in the previous step was performed by performing twice a cycle of removing the Fmoc group of the α-amino group by the method described above and then adding a 0.3 M chloroacetic acid solution in DMF (8.4 equivalents), 0.28 M HATU solution in DMF (7.8 equivalents), and 1.05 M DIPEA solution in DMF (16.8 equivalents) to the solid-phase resin and shaking the resultant mixture at 25°C for 30 minutes. Deprotection of the side chain and cutting the side chain from the solid-phase resin were performed by the following method. First, the resin obtained after the chloroacetyl group introduction was washed five times with DMF and three times with methylene chloride, and then dried under reduced pressure. A reagent cocktail-A (a mixture of TFA/H₂O/TIS/DODT in a volume ratio of 92.5 : 2.5 : 2.5 : 2.5) was then added to the reaction container containing the solid-phase resin and shaken at 25°C for 60 minutes. A reaction solution was collected from a frit by filtration. The solid-phase resin remaining in the reaction container was shaken again with a cocktail for cutting out the solid-phase resin, and the solution component was collected from the frit and mixed with the filtrate. When the filtrate was added to an excess amount of diethyl ether/hexane (1/1) solvent mixture cooled at 0°C, a white precipitate was generated. The mixture was centrifuged (8500 rpm, 0°C, 30 seconds), and the solution decanted off. The resulting solid was washed again with a small amount of diethyl ether/hexane cooled at 0°C and dried under reduced pressure. The resulting solid was used in the next cyclization reaction. The cyclization reaction of the peptide was performed by dissolving the peptide in DMSO such that a final concentration of the peptide reached 2.5 mM based on the number of moles of the solid-phase resin, then adding 10 equivalents of triethylamine, and shaking the resultant mixture at 25°C for 16 hours. The resultant reaction solution was concentrated under reduced pressure using EZ-2 Elite.

The resultant mixture was subjected to solid phrase extraction using an ASPEC (registered trademark) C18 cartridge manufactured by Gilson. The resultant extract was concentrated under reduced pressure using EZ-2 Elite.

The concentrated extract was analyzed by LC-MS, and in the mass spectrum, the target product was observed in one of main peaks.

Analysis conditions: Retension time = 1.73 min; Column: Kinetex (registered trademark) EVO C18, 1.7 µm, 2.1 mm x 50 mm, 100 Å; Mobile phase: A= 0.025% TFA in H₂O, B = 0.025% TFA in MeCN; Temperature: 60°C; Gradient (B conc (%)): 5% to 95% over 2.10 min, then 95% to 95% over 0.75 min; Flow rate: 0.6 mL/min

ESI-MS(+) Observed value m/z = 1725.0(M + 3H)³⁺, Theoretical value m/z = 5170.91

### [Example 3-2]

The peptide complexes listed in Table 4 were synthesized in the same manner as the synthesis of the peptide complexes shown below unless there were other synthesis examples. The ESI-MS (m/z) in Table 4 indicates the ESI-MS (+) observed value, and [M+XH]X+ indicates the value of X when the number of protons added in that case is shown as (M+XH)X+.

### Synthesis of GFs_c-Met-0012 complex (GFs_c-Met-0012-PEG12 dimer; peptide complex No. 17 in Table 4)

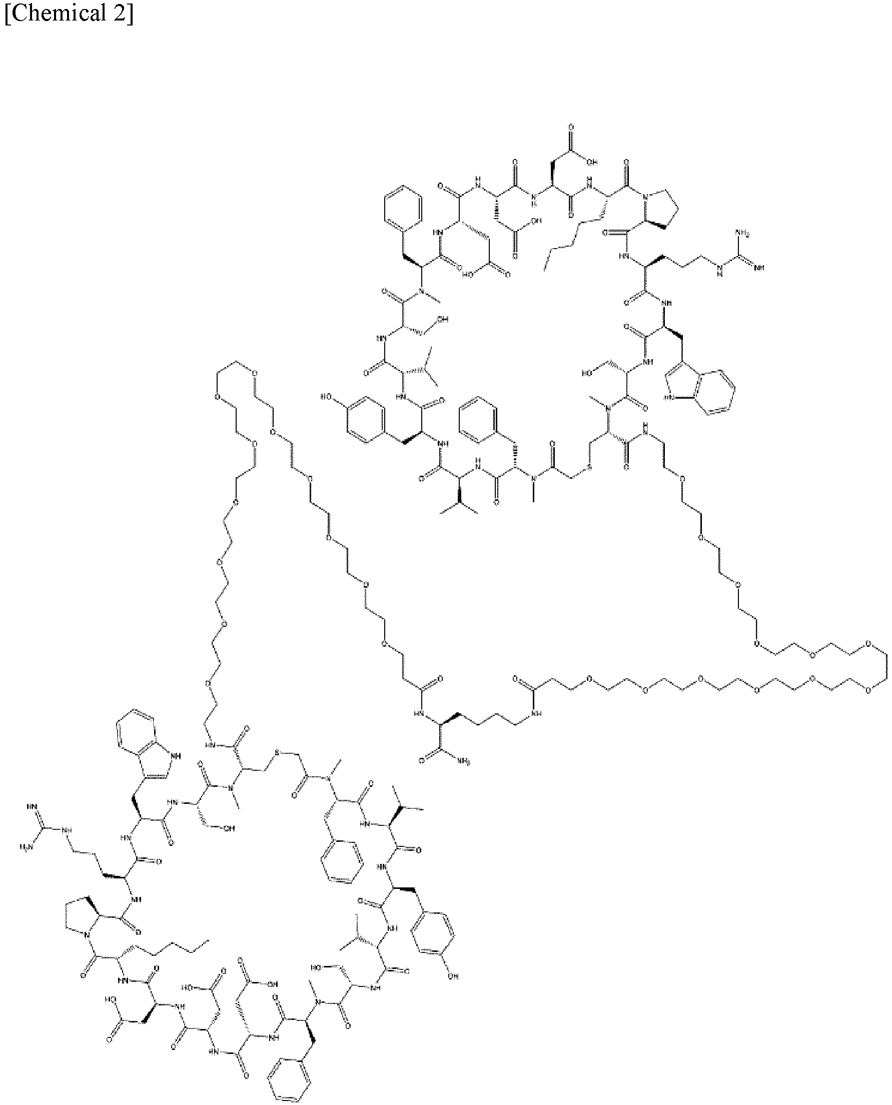

A target peptide was synthesized using Sieber amide resin (Watanabe Chemical Industries, 0.47 mmol/g, 0.11 g), starting with removal of the Fmoc group by the general method described above. The synthesis was performed using Siro I available from Biotage as a solid-phase synthesizer, following the manufacturer's manual. The synthesis was performed by after introducing Fmoc-Lys(Fmoc)-OH into the solid-phase resin, removing both Fmoc groups and the simultaneously elongating from the two amino groups on Lys. For the introduction of each residue, Fmoc-AA/HATU/DIPEA (8.4 equivalents/8 equivalents/16 equivalents) was used per equivalent of resin. For Fmoc removal, Fmoc was reacted with a 0.1M HOBt containing 5% piperidine solution in DMF at 25°C for 5 minutes, then the solution was removed, and a 0.1M HOBt containing 5% piperidine solution in DMF was added again and reacted for 15 minutes. Introduction of a chloroacetyl group into the solid-phase resin containing the Fmoc-protected peptide obtained in the previous step was performed by removing the Fmoc group of the α-amino group by the method described above, and then adding a 0.5 M chloroacetic acid solution in DMF (10 equivalents), 0.49 HATU solution in DMF (9.8 equivalents), and 0.5 M DIPEA solution in DMF (10 equivalents) to the solid-phase resin and shaking the resultant mixture at room temperature for 30 minutes. Deprotection of the side chain and cutting the side chain from the solid-phase resin were performed by the following method. First, the resin obtained after the chloroacetyl group introduction was washed five times with DMF and three times with methylene chloride, and then dried under reduced pressure. A reagent cocktail-A (a mixture of TFA/H₂O/TIS/DODT in a volume ratio of 92.5 : 2.5 : 2.5 : 2.5) was then added to the reaction container containing the solid-phase resin and shaken at 25°C for 60 minutes. A reaction solution was collected from a frit by filtration. The solid-phase resin remaining in the reaction container was shaken again with a cocktail for cutting out the solid-phase resin, and the solution component was collected from the frit and mixed with the filtrate. When the filtrate was added to an excess amount of diethyl ether/hexane (1/1) solvent mixture cooled at 0°C, a white precipitate was generated. The mixture was centrifuged (9000 rpm, 2 minutes), and the solution decanted off. The resulting solid was washed again with a small amount of diethyl ether/hexane cooled at 0°C and dried under reduced pressure. The resulting solid was used in the next cyclization reaction. The cyclization reaction of the peptide was performed by dissolving the peptide in DMSO/acetonitrile/water (18/1/1) such that a final concentration of the peptide reached 1 mM based on the number of moles of the solid-phase resin, then adding 20 equivalents of triethylamine, and shaking the resultant mixture at 25°C for 15 hours. The resultant reaction solution was concentrated under reduced pressure using EZ-2 Elite.

The resulting mixture was purified under the following conditions: (Column: Waters XSelect (registered trademark) C18 19 mm x 150 mm; Mobile phase: A = 0.1% TFA in H₂O, B = 0.1% TFA in MeCN; Temperature: 40°C; Gradient (B conc (%)): 13% to 38% over 3 min, then 38% to 43% over 8 min, then 43% to 60% over 1 min; Flow rate: 17 mL/min.

The purity of the target product calculated from an area ratio in LC/MS (UV wavelength: 225 nm) chromatogram under the following analysis conditions was 59.97%.

Analysis conditions: Retension time = 5.75 min; Column: Kinetex (registered trademark) EVO C18, 2.6 µm, 2.1 mm x 150 mm, 100 Å; Mobile phase: A = 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min

ESI-MS(+) Observed value m/z = 1300.6(M + 4H)⁴⁺, Theoretical value m/z = 5196.56

### EXAMPLE 4

### Measurement of c-Met Agonist Activity in Peptide Complex of the Present Invention in Phospho-c-Met AlphaLISA Assay

To evaluate the activation potency (c-Met agonist activity) of c-Met of the peptide complex of the present invention, the phosphorylation of c-Met was verified.

Human A431 cells were cultured in DMEM high glucose, GlutaMAX (registered trademark) Supplement, pyruvate (Thermo Fisher Scientific), containing 10% FBS (Gibco). After cells were detached using Accutase (Innovative cell technologies), they were seeded into a 96-well plate at 10000 cells per well and cultured overnight. The next day, for starvation, the culture medium was changed to DMEM high glucose, GlutaMAX (registered trademark) Supplement, pyruvate (Thermo Fisher Scientific), containing 0.1% BSA (Sigma-Aldrich), and the cells were cultured overnight. Then, the peptide complex synthesized in Example 3 or Recombinant Human HGF Protein (R&D systems) as a control was added, and after 15 minutes of stimulation, cells were lysed in a Lysis Buffer provided in the AlphaLISA (registered trademark) SureFire (registered trademark) Ultra^{™} Phospho -c-Met (Tyr1234/1235) kit (PerkinElmer). The assay was performed according to the kit protocol, and a SpectraMax (registered trademark) Paradigm (registered trademark) multimode microplate reader (Molecular Devices, LLC.) was used for signal detection.

The obtained signals were analyzed with GraphPad Prism (MDF Co., Ltd.), and the maximum value of the signal induced by HGF was set as 100%, and those showing more than 50% activation were described as 2 and those showing less than 50% activation as 1. Table 3 shows the results. In the same manner, activity measurement was performed using the same monomeric peptide, but it did not show c-Met agonist activity.

In the same manner, the C50 value was calculated by GraphPad Prism as the concentration of peptide showing 50% activity of the signal induced by HGF. Table 4 shows the results. In this assay, the value of EC50 of HGF, which was a control, was 0.66 nM.

Further, for a complex (GFs_c-Met-00014336-PEG13 dimer; complex no. 49 in Table 4) of a peptide (SEQ ID NO: 34) and a linker (SEQ ID NO: 37), a graph of c-Met agonist activity is shown in FIG. 1. The EC50 of the peptide complex was 0.72 nM, showing the c-Met agonist activity almost the same as that of HGF.

These results indicated that the peptide complex of the present invention certainly had c-Met agonist activity.

### EXAMPLE 5

### Evaluation of HUVEC Cell Proliferation

In order to evaluate the biological activity of the peptide complex of the present invention, the cell proliferation inducing activity was verified.

Normal human umbilical vein vascular endothelial cells (HUVECs) were cultured using HuMedia-EG2 (KURABO INDUSTRIES LTD.). After cells were detached using Accutase (Innovative cell technologies), they were resuspended in a Medium without serum or growth factors (Cell system) containing 5% FBS (MBL), seeded into a 96-well plate at 10000 cells per well, and cultured overnight. The next day, a complex (GFs_c-Met-00014336-PEG13 dimer; peptide complex no. 49 in Table 4) of the peptide (SEQ ID NO: 34) synthesized in Example 2 and a linker (SEQ ID NO: 37), or Recombinant Human HGF Protein (R&D systems) as a control was added and cultured for another 2 days. The medium was then removed and the cell count was determined quantitatively using the CellTiter-Glo (registered trademark) Luminescent Cell Viability Assay reagent (Promega) according to the protocol provided. A SpectraMax (registered trademark) Paradigm (registered trademark) multimode microplate reader (Molecular Devices, LLC.) was used for detection.

The obtained signal value was analyzed by GraphPad Prism, and EC50 was calculated. A graph of the activity obtained is shown in FIG. 2. As a result, the EC50 of HGF was 0.37 nM and the EC50 of the peptide complex was 0.55 nM, indicating that the peptide complex of the present invention showed c-Met agonist activity almost the same as that of HGF.

These results indicated that the peptide complex of the present invention certainly had activity to induce proliferation of human cells.

### EXAMPLE 6

The following peptide complex was synthesized.

### [Example 6-1]

### Synthesis of Cyclic Peptide having Amino Acid Sequence of SEQ ID NO: 34 in Table 1

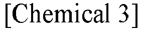

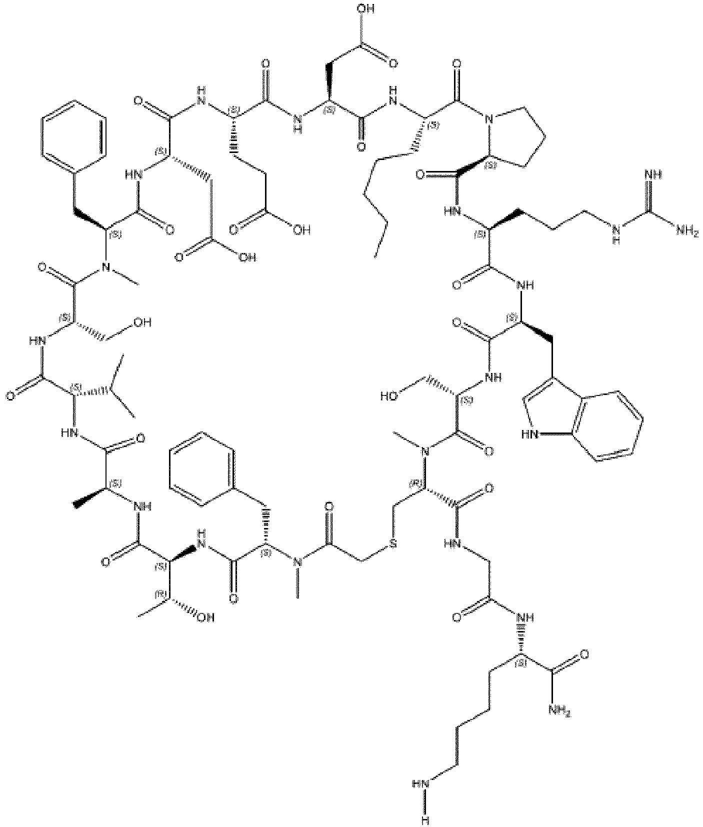

A target peptide was synthesized using Sieber amide resin (Watanabe Chemical Industries, 0.65 mmol/g, 0.31 g), starting with removal of the Fmoc group by the general method described above. The synthesis was performed using Siro I available from Biotage as a solid-phase synthesizer, following the manufacturer's manual. For the introduction of each residue, Fmoc-AA/HATU/DIPEA(3.2 equivalents/3 equivalents/6.3 equivalents) was used per equivalent of resin. For Fmoc removal, Fmoc was reacted with a 20% piperidine solution in DMF at 25°C for 5 minutes, then the solution was removed, and a 20% piperidine solution in DMF was added again and reacted for 15 minutes. Introduction of a chloroacetyl group into the solid-phase resin containing the Fmoc-protected peptide obtained in the previous step was performed by removing the Fmoc group of the α-amino group by the method described above, and then adding a 0.5 M chloroacetic acid solution in DMF (5 equivalents), 0.49 HATU solution in DMF (4.9 equivalents), and 0.5 M DIPEA solution in DMF (5 equivalents) to the solid-phase resin and shaking the resultant mixture at room temperature for 30 minutes. Deprotection of the side chain and cutting the side chain from the solid-phase resin were performed by the following method. First, the resin obtained after the chloroacetyl group introduction step was washed five times with DMF and three times with methylene chloride, and then dried under reduced pressure. A reagent cocktail-A (a mixture of TFA/H₂O/TIS/DODT in a volume ratio of 92.5 : 2.5 : 2.5 : 2.5) was then added to the reaction container containing the solid-phase resin and shaken at 25°C for 60 minutes. A reaction solution was collected from a frit by filtration. The solid-phase resin remaining in the reaction container was shaken again with a cocktail for cutting out the solid-phase resin, and the solution component was collected from the frit and mixed with the filtrate. When the filtrate was added to an excess amount of diethyl ether/hexane (1/1) solvent mixture cooled at 0°C, a white precipitate was generated. The mixture was centrifuged (9000 rpm, 2 minutes), and the solution decanted off. The resulting solid was washed again with a small amount of diethyl ether/hexane cooled at 0°C and dried under reduced pressure. The resulting solid was used in the next cyclization reaction. The cyclization reaction of the peptide was performed by dissolving the peptide in DMSO/acetonitrile/water (18/1/1) such that a final concentration of the peptide reached 2.5 mM based on the number of moles of the solid-phase resin, then adding 10 equivalents of triethylamine, and shaking the resultant mixture at 25°C for 4 hours. The resultant reaction solution was concentrated under reduced pressure using EZ-2 Elite.

The resultant crude product was purified under the following conditions: (Column: Waters Xbridge (registered trademark) C18 5 µm, 50 mm x 150 mm; Mobile phase: A= 0.1% TFA in H₂O, B = 0.1% TFA in MeCN; Temperature: 40°C; Gradient (B conc (%)): 5% to 30% over 3 min, then 30% to 35% over 8 min, then 35% to 60% over 1 min; Flow rate: 120 mL/min.

The purity of the target product calculated from an area ratio in LC/MS (UV wavelength: 225 nm) chromatogram under the following analysis conditions was 96.90%.

Analysis conditions: Retention time = 3.84 min; Column: Kinetex (registered trademark) EVO C18, 2.6 µm, 2.1 mm x 150 mm, 100 Å; Mobile phase: A = 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min
ESI-MS(+) Observed value m/z = 1027.7(M + 2H)²⁺

The resultant cyclic peptide was used for the synthesis of peptide complex

### [Example 6-2]

### Synthesis of GFs_c-Met-00014336 (GFs_c-Met-00014336-PEG13 dimer; peptide complex No. 49 in Table 4)

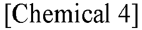

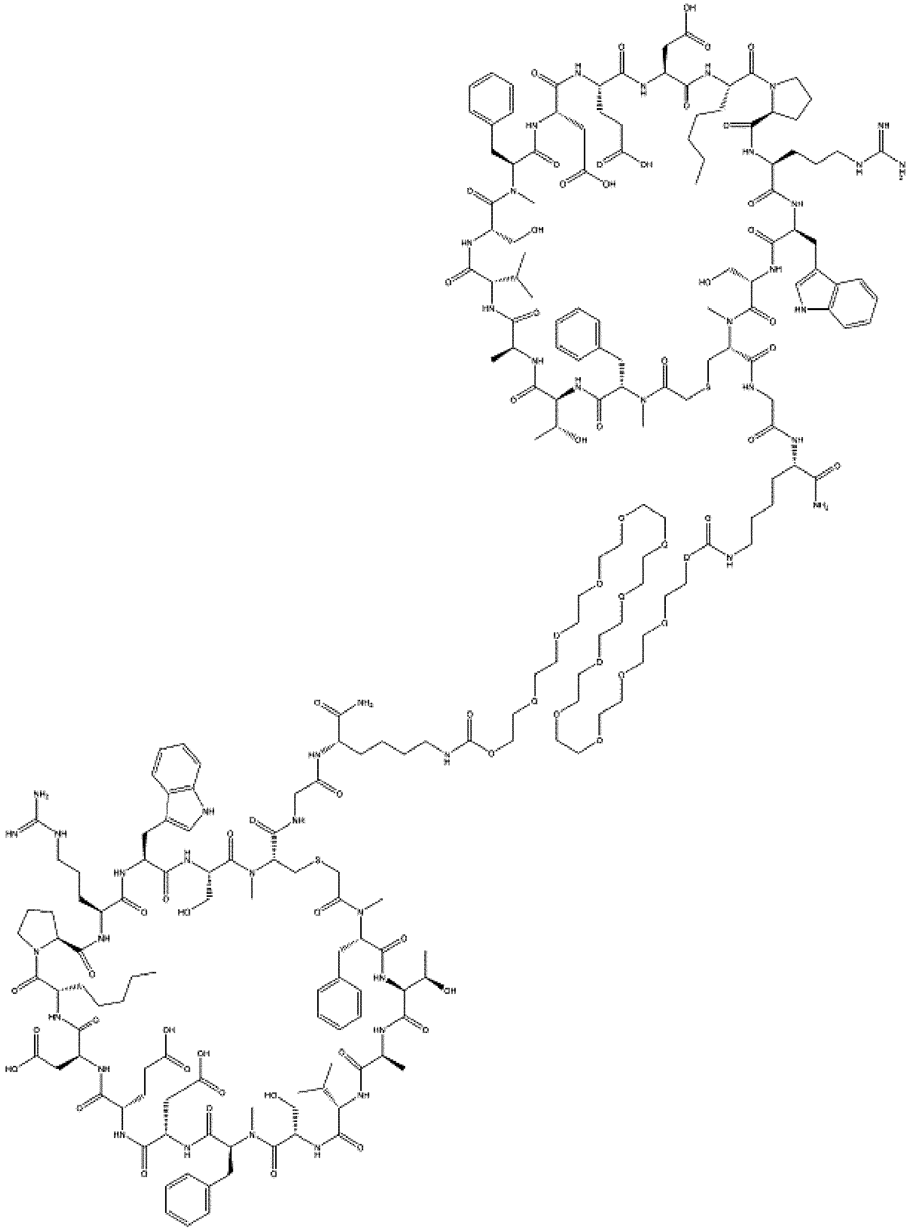

GFs_c-Met-00014234 (8.0 mg, 3.51 µmol) was dissolved in DMF (0.2 mL), a solution (23 µL) of DIPEA (2.3 µL, 13 µmol) in DMF and a solution (14 µL)of bis(2,5-dioxopyrrolidin-1-yl)(3,6,9,12,15,18,21,24,27,30,33-undecaoxapentatriacontan-1,35-diyl)biscarbonate (1.38 mg, 1.67 µmol) in DMF were added thereto, and the mixture was stirred at 25°C for 16 hours.

The resulting mixture was purified under the following conditions: (Column: Waters XSelect (registered trademark) C18 19 mm x 150 mm; Mobile phase: A = 0.1% TFA in H₂O, B = 0.1% TFA in MeCN; Temperature: 40°C; Gradient (B conc (%)): 13% to 38% over 3 min, then 38% to 43% over 8 min, then 43% to 60% over 1 min; Flow rate: 17 mL/min.

The purity of the target product calculated from an area ratio in LC/MS (UV wavelength: 225 nm) chromatogram under the following analysis conditions was 96.42%.

Analysis conditions: Retention time = 5.07 min; Column: Kinetex (registered trademark) EVO C18, 2.6 µm, 2.1 mm x 150 mm, 100 Å; Mobile phase: A = 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min
ESI-MS(+) Observed value m/z = 1177.2(M + 4H)⁴⁺, Theoretical value m/z = 4703.25

### [Example 6-3]

### Synthesis of GFs c-Met-00014305 (GFs c-Met-00014305-PEG17 dimer; peptide complex No. 50 in Table 4)

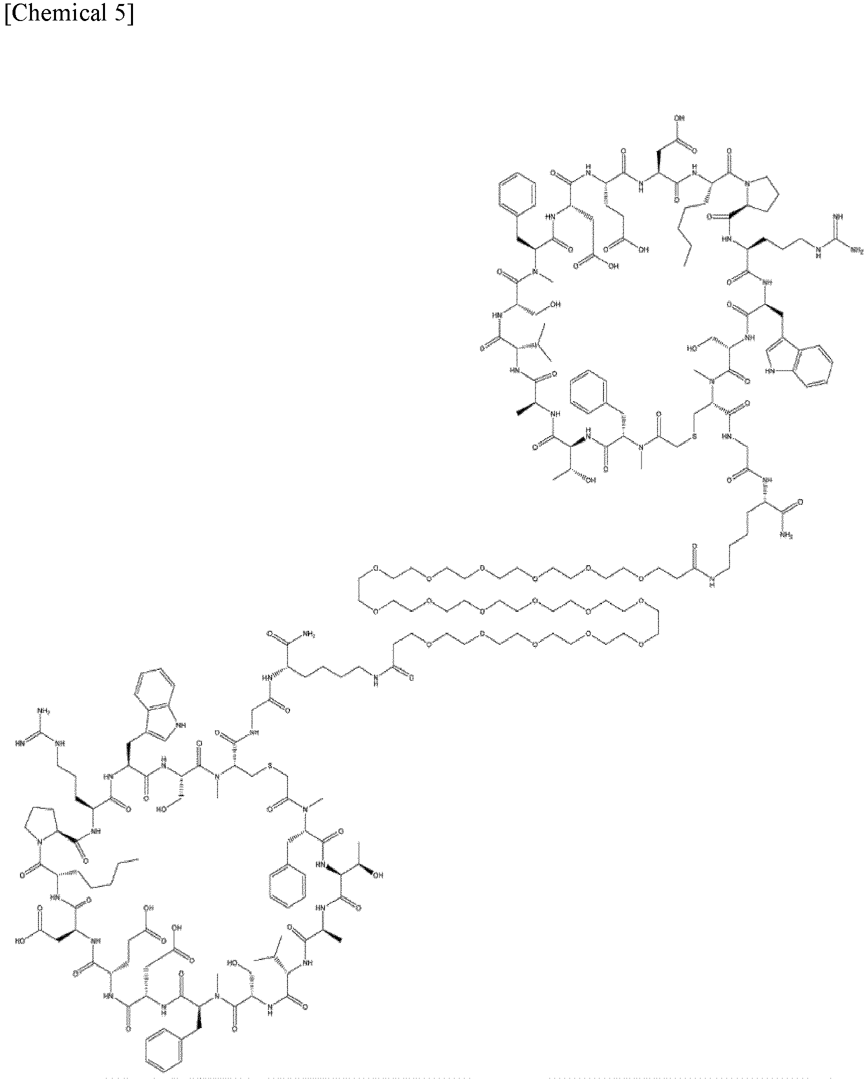

The cyclic peptide (GFs_c-Met-00014234) (8.0 mg, 3.51 µmol) synthesized in Example 6-1 was dissolved in DMF (0.25 mL), a solution (20 µL) of triethylamine (2.0 µL, 14 mmol) in DMF and a solution (18 µL) of bis(2,5-dioxopyrrolidin-1-yl)4,7,10,13,16,19,22,25,28,31,34,37,40,43,46,49,52 - heptadecaoxapentapentacontanedioate (1.75 mg, 1.65 µmol) in DMF were added thereto, and the mixture was stirred at 25°C for 16 hours.

The resulting mixture was purified under the following conditions: (Column: Waters XSelect (registered trademark) C18 5 µm, 19 mm x 150 mm; Mobile phase: A= 0.1% TFAin H₂O, B = 0.1% TFAin MeCN; Temperature: 40°C; Gradient (B conc (%)): 12% to 37% over 3 min, then 37% to 42% over 8 min, then 42% to 60% over 1 min; Flow rate: 17 mL/min.

The purity of the target product calculated from an area ratio in LC/MS (UV wavelength: 225 nm) chromatogram under the following analysis conditions was 94.19%.

Analysis conditions: Retention time = 5.92 min; Column: Kinetex (registered trademark) EVO C18, 2.6 µm, 2.1 mm x 150 mm, 100 Å; Mobile phase: A = 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min
ESI-MS(+) Observed value m/z = 1235.3(M + 4H)⁴⁺, Theoretical value m/z = 4935.41

### [Example 6-4]

### Synthesis of GFs_c-Met-00014318 (GFs _c-Met-00014318-PEG9 dimer; peptide complex No. 51 in Table 4)

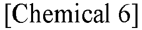

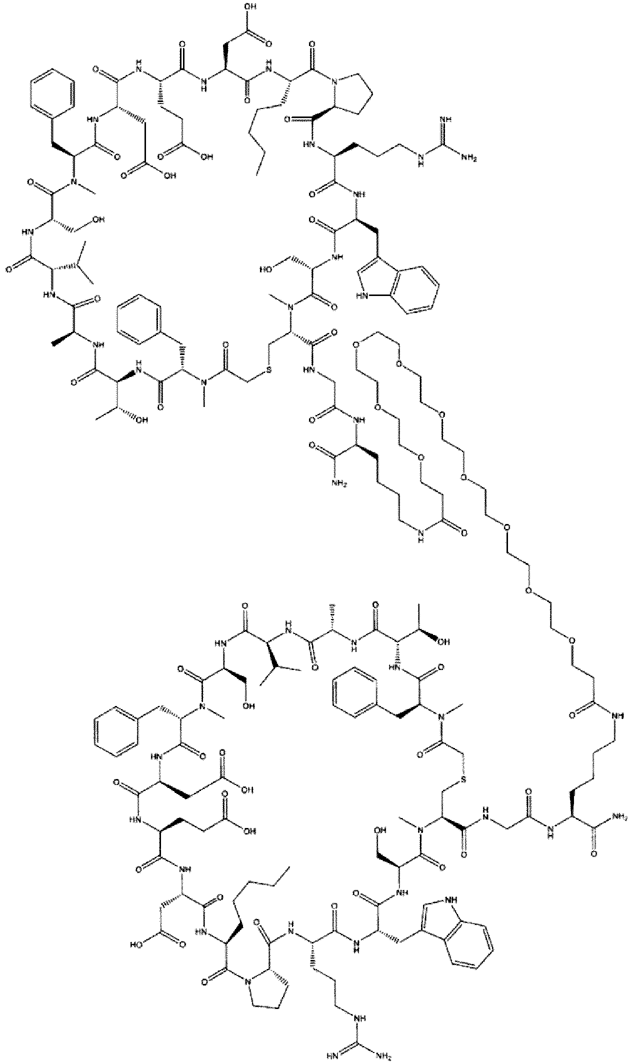

GFs_c-Met-00014234 (8.0 mg, 3.51 µmol) was dissolved in DMF (0.25 mL), a solution (20µL) of triethylamine (2.0 µL, 14 µmol) in DMF and a solution (12 µL) of bis(2,5- dioxopyrrolidin-1-yl)4,7,10,13,16,19,22,25,28-nonaoxahene triacontanedioate (1.17 mg, 1.65 µmol) in DMF were added thereto, and the mixture was then stirred at 25°C for 16 hours.

The resulting mixture was purified under the following conditions: (Column: Waters XSelect (registered trademark) C18 19 mm x 150 mm; Mobile phase: A = 0.1% TFA in H₂O, B = 0.1% TFA in MeCN; Temperature: 40°C; Gradient (B conc (%)): 11% to 36% over 3 min, then 36% to 41% over 8 min, then 41% to 60% over 1 min; Flow rate: 17 mL/min.

The purity of the target product calculated from an area ratio in LC/MS (UV wavelength: 225 nm) chromatogram under the following analysis conditions was 94.56%.

Analysis conditions: Retention time = 5.03 min; Column: Kinetex (registered trademark) EVO C18, 2.6 µm, 2.1 mm x 150 mm, 100 Å; Mobile phase: A = 0.025% TFA in H₂O, B = 0.025% TFA in MeCN; Temperature: 60°C; Gradient (B conc (%)): 20% to 60% over 7.15 min, then 60% to 95% over 0.30 min, then 95% to 95% over 1.55 min; Flow rate: 0.5 mL/min
ESI-MS(+) Observed value m/z = 1147.1(M + 4H)⁴⁺, Theoretical value m/z = 4582.2

### EXAMPLE 7

### Evaluation of Tube formation

Renal proximal tubule epithelial cells (RPTEC) were cultured in a growth medium (Renal Epithelial Cell Basal Medium (REBM) + 0.5% FBS + 2.4 mM L-Alanyl-L-Glutamine + 10 nM Triiodothyronine + 10 ng/mL rh EGF + 100 ng/mL Hydrocortisone Hemisuccinate + 5 µg/mL rh Insulin + 1 µM Epinephrine + 5 ug/mL Transferrin) (ATCC). The cells were detached using Trypsin-EDTA, and then resuspended in an assay medium (REBM + 0.5% FBS + 2.4 mM L-Alanyl-L-Glutamine + 10 nM Triiodothyronine + 100 ng/mL Hydrocortisone Hemisuccinate + 1 µM Epinephrine + 5 µg/mL Transferrin + 1.8 mM CaCl2).

RPTECs were resuspended in a mixture of Cellmatrix (registered trademark) Type I-A (Nitta Gelatin Inc.), concentrated culture solution (Nitta Gelatin Inc.) and a reconstitution buffer solution (Nitta Gelatin Inc.) on ice according to the instructions. 300 uL of each resultant mixture was added to each well of 48-well plate at 200000 cells per well. The 48-well plate was stood still in a CO2 incubator (37°C, 5% CO2) for 60 minutes to gelate the suspension.

To the assay medium, 2 nM HGF, 1.6 nM EGF or a complex GFs_c-Met-00014336-PEG13 dimer; peptide complex No. 49 in Table 4) of a peptide (SEQ ID NO: 34) synthesized in Example 2 and a linker (SEQ ID NO: 37) at 0.08, 0.4, 2, and 10 nM, was added and 400 µL of each resultant mixture was added to each well of 48-well plate to which the gelated suspension had been added as mentioned above. The 48-well plate was stood still in a CO2 incubator (37°C, 5% CO2), and the medium was changed every 3 days. The presence or absence of tube formation was observed and evaluated using a phase-contrast microscope on the third day and the eighth day of the start of the culturing.

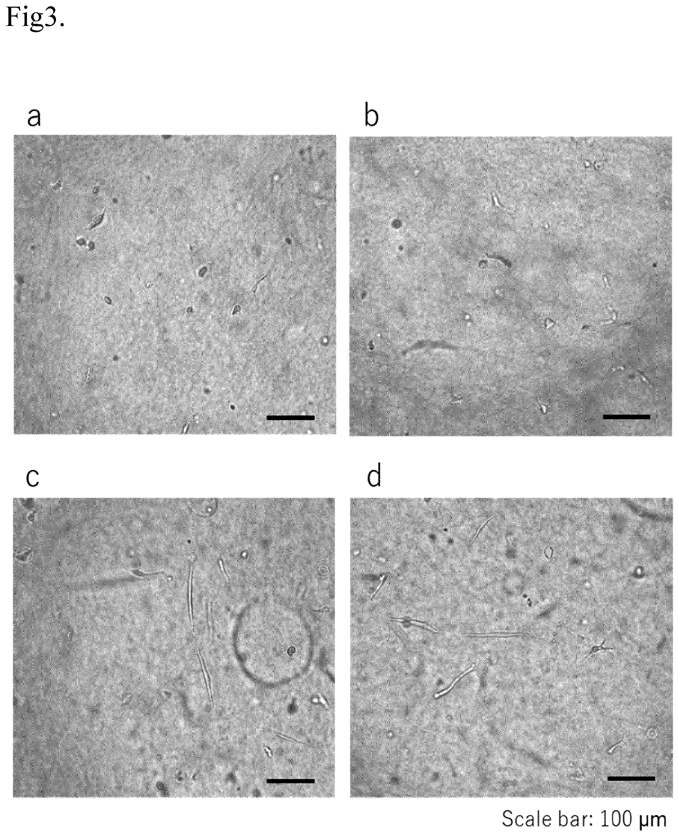

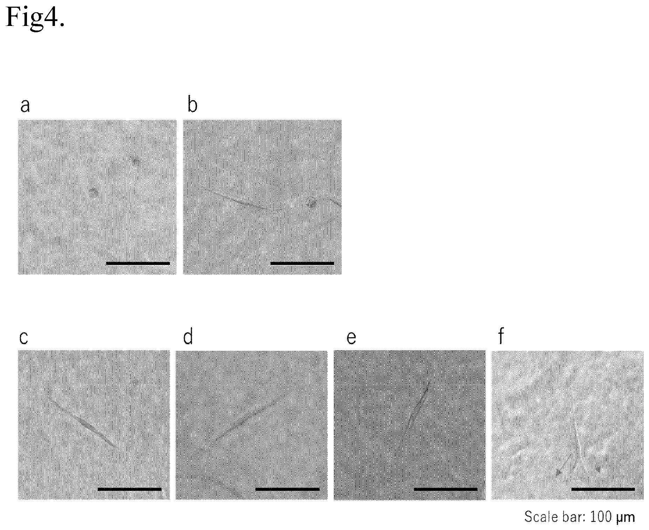

The results are shown in FIGS. 3 and 4. FIG. 3 shows the results on the third day, a is an observed result of wells of MOCK (no addition), b is of wells to which 1.6 nM EGF was added, c is of wells to which 2 nM HGF was added, and d is of wells to which 2 nM peptide complex (peptide complex No. 49 in Table 4) was added. FIG. 4 shows the results on the eighth day, a is an observed result of wells of MOCK (no addition), b is of wells to which 2 nM HGF was added, c is of wells to which 0.08 nM of a complex (GFs_c-Met -00014336-PEG13 dimer; peptide complex No. 49 in Table 4) of peptide (SEQ ID NO: 34) synthesized in Example 2 and a linker (SEQ ID NO: 37) was added, d is of wells to which 0.4 nM of the complex was added, e is of wells to which 2 nM of the complex was added, and f is of wells to which 10 nM of the complex was added.

These results indicated that the peptide complex of the present invention promoted tube formation as in HGF. It was shown in f of FIG. 4 that not only tube formation but also branch formation was promoted. Since tube formation requires multifunctional actions such as cell proliferation, migration, and collagen degradation, the peptide complex of the present invention was shown to have physiological activity similar to that of HGF.

### EXAMPLE 8

### Human Phospho-RTK Array Assay

A431 cells were cultured in DMEM high glucose, GlutaMAX (registered trademark) Supplement, pyruvate (Thermo Fisher Scientific), containing 10% FBS (Gibco). After cells were detached using Accutase (registered trademark) (Innovative cell technologies), they were seeded into a 6-well plate at 1000000 cells per well and cultured overnight.

The next day, for starvation, the culture medium was changed to DMEM high glucose, GlutaMAX (registered trademark) Supplement, pyruvate (Thermo Fisher Scientific), containing an assay medium (0.1% BSA (Sigma-Aldrich)), and the cells were cultured overnight. Then, 7.8 nM Recombinant Human HGF Protein (R&D systems) or 2 nM complex (GFs_c-Met 00014336-PEG9 dimer; peptide complex No. 51 in Table 4) of peptide (SEQ ID NO: 34) synthesized in Example 2 and a linker (SEQ ID NO: 36) was added, and after 10 minutes of stimulation, cells were lysed in a Lysis Buffer provided in the Proteome Profiler Human Phospho-RTK Array Kit (R & D systems). Thereafter, the procedure was performed according to the protocol of the kit, and chemiluminescent signals by WesternSure (registered trademark) PREMIUM chemiluminescent substrate kit (LI-COR) were detected using C-DiGit (registered trademark) (Scrum).

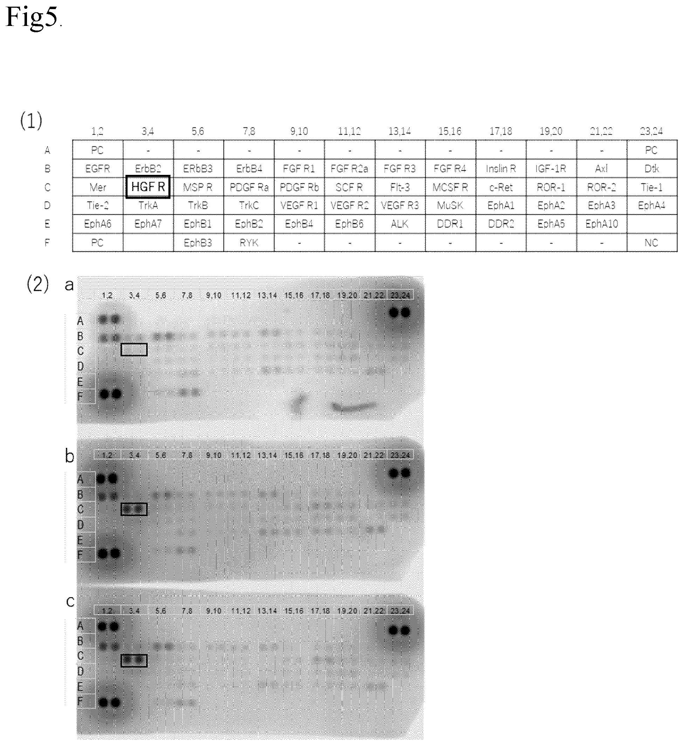

Table 5 shows the results. In (2) in FIG. 5, a is the result of no addition, b is of addition of HGF, and c of addition of the peptide complex. Only HGF R was specifically phosphorylated when the peptide complex was added and when HGF was added. Thus, the peptide complex of the present invention was shown to have a specific phosphorylation pattern similar to that of HGF.

### INDUSTRIAL APPLICABILITY

The present invention may be used in the pharmaceutical and biotechnology industries.

### [Sequence listing]

21-068P_ST25.txt

## Claims

1. A peptide complex comprising
a peptide A that binds to a c-Met protein, wherein
the peptide A is a peptide consisting of an amino acid sequence represented by X¹-X²-X³-V-S-X⁴-D-X⁵-D-X⁶-P-R-W-X⁷-MeC (SEQ ID NO: 1) or an amino acid sequence with substitution, deletion, addition, or insertion of one to three amino acids in the amino acid sequence represented by SEQ ID NO: 1,
X¹ is an amino acid that is optionally N-alkylated,
X² is any amino acid,
X³ is any amino acid,
X⁴ is a hydrophobic amino acid that is optionally N-alkylated,
X⁵ is any amino acid,
X⁶ is an amino acid having an alkyl chain in the side chain that is optionally substituted, or S, and
X⁷ is any amino acid.

2. The peptide complex according to claim 1, wherein
X¹ is A that is optionally N-methylated or F that is optionally N-methylated,
X² is a hydrophilic amino acid, an aliphatic, branched amino acid, or an aromatic amino acid,
X³ is a hydrophilic amino acid, an aliphatic amino acid, or an aromatic amino acid,
X⁴ is a hydrophobic amino acid that is optionally N-methylated,
X⁵ is a hydrophilic amino acid, an aliphatic, branched amino acid, or P, and
X⁷ is a hydrophilic amino acid or an aliphatic amino acid.

3. The peptide complex according to claim 1, wherein
X¹ is A that is optionally N-methylated or F that is optionally N-methylated,
X² isV, T, E, Q, orW,
X³ is A, R, Y, or D,
X⁴ is F that is optionally methylated or L that is optionally methylated,
X⁵ is D, E, S, P, or V,
X⁶ is an (S)-2-aminoheptanoic acid (Ahp), an R, L-norleucine (Nle), an (S)-2,7-diaminoheptanoic acid (Hty), or S, and
X⁷ is S, A, an L-α-aminobutanoic acid (Abu), D, Q, or V.

4. The peptide complex according to claim 1, wherein
the peptide complex comprises:
a first peptide;
a second peptide; and
a linker connecting the first peptide and the second peptide, and
at least one of the first peptide or the second peptide is the peptide A.

5. The peptide complex according to claim 4, wherein
the first peptide and the second peptide are identical to or different from each other, and are each the peptide A.

6. The peptide complex according to claim 5, wherein
the peptide A is a peptide consisting of an amino acid sequence represented by MeF-T-A-V-S-MeF-D-E-D-Ahp-P-R-W-S-MeC (SEQ ID NO: 34) or an amino acid sequence with substitution, deletion, addition, or insertion of one to three amino acids in the amino acid sequence represented by SEQ ID NO: 34, and is a peptide that binds to the c-Met protein.

7. The peptide complex according to claim 6, wherein
the peptide A is a cyclic peptide.

8. The peptide complex according to claim 7, wherein
the first peptide and the second peptide are the same peptide A, and have a C-terminal bound to the linker.

9. The peptide complex according to claim 8, wherein
the peptide A is a peptide consisting of an amino acid sequence represented by any of SEQ ID NOs: 2 to 34.

10. The peptide complex according to claim 9, wherein
the linker is a PEG linker.

11. The peptide complex according to claim 9, wherein
the linker is consisting of a sequence represented by any of SEQ ID NOs: 35 to 41 or a sequence with substitution, deletion, addition, or insertion of one to three amino acids in the sequence represented by any of SEQ ID NOs: 35 to 41.

12. A c-Met protein agonist comprising the peptide complex according to claim 11.

13. A pharmaceutical composition comprising:
the peptide complex according to any one of claims 5 to 12; and
a pharmaceutically acceptable carrier.

14. The pharmaceutical composition according to claim 13, wherein
the pharmaceutical composition is used to treat or prevent a disease selected from the group consisting of ischemic heart disease, acute hepatitis, fulminant hepatitis, hepatic cirrhosis, biliary atresia, fatty liver, acute renal failure, chronic renal failure, diabetic nephropathy, acute pneumonia, pulmonary fibrosis, vascular disease, myocardial infarction, dilated cardiomyopathy, skin ulcer, cerebral infarction, arteriosclerosis obliterans, gastric ulcer, and amyotrophic lateral sclerosis.

15. A culture medium additive comprising the peptide complex according to any one of claims 1 to 11.

16. The culture medium additive according to claim 15, wherein
the culture medium additive is used to culture cells or tissues derived from human.
